Europäisches Patentamt
European Patent Office
Office européen des brevets

(11) Publication number: **0 312 158**
**A2**

(12) # EUROPEAN PATENT APPLICATION

(21) Application number: 88202211.4

(51) Int. Cl.4: C07K 5/02 , A61K 37/64

(22) Date of filing: 05.10.88

(30) Priority: 13.10.87 US 107213

(43) Date of publication of application: 19.04.89 Bulletin 89/16

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Chakravarty, Prasun K.
16 Churchill Road
Edison, New Jersey 08820(US)
Inventor: Schoen, William
196 White Birch Road
Edison, New Jersey 08837(US)
Inventor: Parsons, William H.
2171 Oliver Street
Rahway New Jersey 07065(US)
Inventor: Greenlee, William J.
115 Herrick Avenue
Teaneck New Jersey 07666(US)

(74) Representative: Hesketh, Alan, Dr.
European Patent Department Merck & Co., Inc. Terlings Park Eastwick Road
Harlow Essex, CM20 2QR(GB)

(54) **Tetrapeptide renin inhibitors having novel c-terminal amino acid amide.**

(57) Tetrapeptides enzyme inhibitors of the formula:

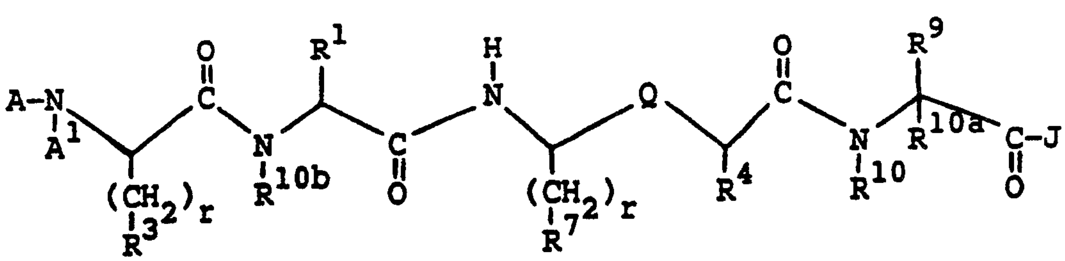

and analogs thereof which inhibit renin and are useful for treating various forms of renin-associated hypertension, hyperaldosteronism and congestive heart failure; compositions containing these renin-inhibitory peptides, optionally with other antihypertensive agents; and methods of treating hypertension, hyperaldosteronism or congestive heart failure or of establishing renin as a causative factor in these problems employing the novel tetrapeptides.

EP 0 312 158 A2

# TETRAPEPTIDE RENIN INHIBITORS HAVING NOVEL C-TERMINAL AMINO ACID AMIDE COMPONENTS

The present invention is concerned with novel peptides which inhibit the angiotensinogen-cleaving action of the proteolytic enzyme, renin, with pharmaceutical compositions containing the novel peptides of the present invention as active ingredients, with methods of treating renin-associated hypertension, hyperaldosteronism, and congestive heart failure, with diagnostic methods which utilize the novel peptides of the present invention, and with methods of preparing the novel peptides of the present invention.

## BACKGROUND OF THE INVENTION

Renin is an endopeptidase (molecular weight about 40,000) produced and secreted by the juxtaglomerular cells of the kidney. Renin has a high specificity for and cleaves the naturally-occurring plasma glycoprotein, angiotensinogen, at only the 10, 11 peptide bond, i.e., between the 10th (Leu) and 11th (Leu) amino acid residues in the equine substrate, as described by Skeggs et al,J. Exper. Med. 1957, 106, 439, or between Leu 10 and Val 11 in the human renin substrate, as elucidated by Tewksbury et al., Circulation 59, 60, Supp. II: 132, Oct. 1979.

This cleavage of its protein substrate, angiotensinogen, by the aspartic proteinase, renin, splits off the decapeptide, angiotensin I, which is thought to be hemodynamically-inactive, but which is converted in the lungs, kidney or other tissue by angiotensinconverting enzyme (ACE) to the potent pressor octapeptide, angiotensin II. Angiotensin II then causes constriction of the arterioles and is also believed to stimulate release of the sodium-retaining hormone, aldosterone, from the adrenal gland, thereby causing a rise in extra cellular fluid volume. Thus, the renin-angiotensin system plays an important role in normal cardiovascular homeostasis and in some forms of elevated blood pressure (hypertension).

Inhibitors of angiotensin I converting enzyme have proven useful in the modulation of the renin-angiotensin system. Consequently, specific inhibitors of the catalytic and rate-limiting enzymatic step that ultimately regulates angiotensin II production, the action of renin on its substrate, have also been sought as effective investigative tools, as well as therapeutic agents in the treatment of hypertension and congestive heart failure.

Renin antibody, pepstatin, phospholipids, and substrate analogs, including tetrapeptides and octa- to tridecapeptides, with inhibition constants ($K_i$) in the $10^{-3}$ to $10^{-6}$M region, have been studied.

Umezawa et al., in J. Antibiot. (Tokyo) 23: 259-262, 1970, reported the isolation of a peptide, pepstatin, from actinomyces that was an inhibitor of aspartyl proteases such as pepsin, cathepsin D, and renin. Gross et al., Science 175:656, 1972, reported that pepstatin reduces blood pressure in vivo after the injection of hog renin into nephrectomized rats. However, pepstatin has not found very wide application as an experimental agent because of its limited solubility and its inhibition of a variety of other acid proteases in addition to renin.

Many efforts have been made to prepare a specific renin inhibitor based on pig renin substrate analogy, since such analogy has been shown to correlate well with and predict human renin inhibitor activity. The octapeptide amino acid sequence extending from histidine-6 through tyrosine-13

```
  6   7   8   9   10  11  12  13
(-His-Pro-Phe-His-Leu-Leu-Val-Tyr-)
```

has been shown to have kinetic parameters essentially the same as those of the full tetradecapeptide renin substrate.

Kokubu et al., Biochem. Pharmacol., 22, 3217-3223, 1973, synthesized a number of analogs of the tetrapeptide found between residues 10 to 13, but while inhibition could be shown, inhibitory constants were only of the order of $10^{-3}$M. Analogs of a larger segment of renin substrate have been also synthesized, e.g., Burton et al., Biochemistry 14: 3892-3898, 1975, and Poulsen et al., Biochemistry 12: 3877-3882, 1973, but a lack of solubility and weak binding (large inhibitory constant) generally resulted.

Modifications to increase solubility soon established that the inhibitory properties of the peptides are markedly dependent on the hydrophobicity of various amino acid residues. These modifications also established that increasing solubility by replacing lipophilic amino acids with hydrophilic isosteric residues can become counter-productive. Other approaches to increasing solubility have also had limited success.

Modifications designed to increase binding to renin have also been made, but here too, with mixed

results.

A series of inhibitors of renin have been disclosed which contain the unnatural amino acid, statine or its analogs: see, e.g., Veber et al, U.S. Patents 4,384,994 and 4,478,826; Evans et al, U.S. Patent 4,397,786; Boger et al, Nature, 1983, 303, 81-84 and U.S. Patents 4,470,971; 4,485,099; 4,663,310 and 4,668,770; Matsueda et al, EP-A 128 762, 152 255; Morisawa et al., EP-A 186 977; Riniker et al, EP-A 111 266; Bindra et al, EP-A 155 809; Stein et al, Fed. Proc. 1986, 45, 869; and Hölzemann et al, German Offenlegungsschrift DE 3438545. Attempting to explain the effect of statine, Powers et al., in Acid Proteases, Structure, Function and Biology, Plenum Press, 1977, 141-157, observed that in pepstatin, statine occupies the space of the two amino acids on either side of the cleavage site of a pepsin substrate and Tang et al., in Trends in Biochem. Sci., 1:205-208 (1976) and J. Biol. Chem., 251:7088-94, 1976, pointed out that the statine residue of pepstatin resembles the transition state for pepsin hydrolysis of peptide bonds.

Renin inhibitors containing other peptide bond isosteres, including a reduced carbonyl isostere have been disclosed by M. Szelke et al, in work described in published European Patent Applications 45 665 and 104 041; in U.S. Patent 4,424,207, and in PCT Int. Appl. WO 84/03044; in Nature, 299, 555 (1982); Hypertension, 4, Supp. 2, 59, 1981; and British Patent 1,587,809. In Peptides, Structure and Function: Proceedings of the Eighth American Peptide Symposium, ed. V. J. Hruby and D. H. Rich, p. 579, Pierce Chemical Co., Rockford, IL., 1983, Szelke et al also showed isosteric substitutions at the Leu-Leu site of cleavage, resulting in compounds with excellent potency. These and other peptide bond isosteres have also been disclosed in Buhlmayer et al in EP-A 144 290 and 184 550; Hester et al, EP-A 173 481; Raddatz, EP-A 161 588; Dann et al, Biochem. Biophys. Res. Commun . 1986, 134, 71-77; Fuhrer et al, EP-A 143 746; Kamijo et al, EP-A 181 110; Thaisrivongs et al, J. Med. Chem., 1985, 28, 1553-1555; Ryono et al., EP-A 181 071; and Evans et al, U.S. Patent 4,609,641.

Other modifications which have been tried include preparing renin inhibitors with non-peptide C termini, such as disclosed in European Published Applications 172 346 and 172 347; Evans et al, J. Med. Chem., 1985, 28, 1755-1756; and Bock et al, Peptides, Structure and Function: Proceedings of the Ninth American Peptide Symposium, ed. C. M. Deber et al, pp.751-754, Pierce Chemical Co., Rockford, IL, 1985. Kokubu et al, in Hypertension, 1985, 7, Suppl. I, p. 8-10 and Matsueda et al, in Chemistry Letters, 1985, 1041-1044 and in European Published Applications 128 762 and 152 255 disclosed peptide aldehyde renin inhibitors, and Hanson et al in Biochem. Biophys. Res. Commun. 1985, 132, 155-161, reported peptide glycol inhibitors.

These various renin inhibitors all generally comprise peptide based inhibitors in which a sequence of the type: ...A-B-D-E-F-G-J-K-L... , where G is a peptide bond mimic and A,B,D,E,F,J,K, and L may individually be absent or may represent naturally-occuring or modified amino acids. Typical sequences of this type include:

```
    7   8   9   10  11  12
...BOC-Pro-Phe-His-Sta-Leu-Phe... ,

       8   9   10  11
or ...BOC-Phe-His-Sta-Leu... ,
```

where the N-terminus typically comprises an amino acid protecting group such as BOC or CBZ, and the N-terminal amino acids are Pro-Phe-His or Phe-His.

It was an object of this invention to prepare lower molecular weight peptides which have enhanced biological potency in inhibiting the renin enzyme. It was also an object to prepare peptides wherein a stabilizing peptide bond mimic is substituted for the 10- and 11- position amino acids in the analogous substrate. It was a further object of this invention to prepare peptides comprising modifications at the C-terminus which constructively alter the solubility and bioavailability of the peptide. It was also an object of this invention to prepare peptides with greater oral bioavailability and longer duration of action. It was still a further object of this invention to prepare novel peptides which are more useful antihypertensive agents, and compounds useful in treating hyperaldosteronism and congestive heart failure.

## DETAILED DESCRIPTION OF THE INVENTION

The present invention discloses renin inhibitory tetrapeptides of the formula:

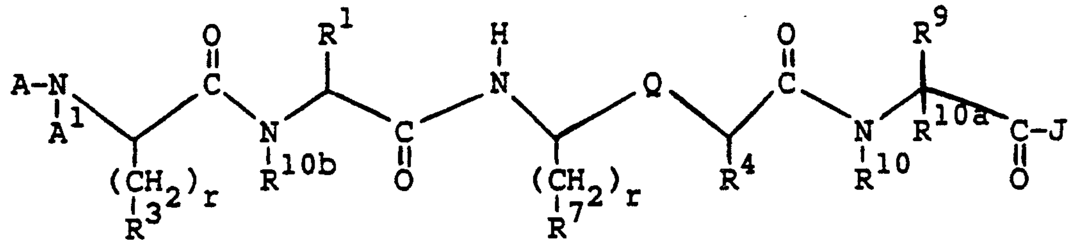

(I),

wherein:

A is hydrogen;

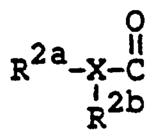

,

where

X is -O-; -O- CH-; - CH-O-; - CH-; N -; -NH- CH-; or -S- CH-; and

$R^{2a}$ and $R^{2b}$ are independently hydrogen; unsubstituted or mono- or disubstituted $C_1$-$C_4$-alkyl, wherein the substituent(s) is/are independently chosen from the group consisting of amino; mono- or di-$C_1$-$C_4$-alkylamino; t-butoxycarbonylamino; carbobenzyloxyamino; acetylamino; trimethylacetylamino; t-butylacetylamino; hydroxy; $-SO_3H$; $-CO_2H$; $-CO_2$-$C_1$-$C_4$-alkyl; $C_1$-$C_4$-alkyl-$SO_2$; aryl, where aryl is unsubstituted or mono-, di- or trisubstituted phenyl or naphthyl, wherein the substituent(s) is/are independently selected from the group consisting of $C_1$-$C_8$-alkyl, amino, mono- or di-$C_1$-$C_4$-alkylamino, amino-$C_1$-$C_4$-alkyl, hydroxy-$C_1$-$C_4$-alkyl, phenyl-$C_1$-$C_4$-alkyl, mono- or di-$C_1$-$C_4$-alkyl-amino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$-alkyl, $CO_2$-$C_1$-$C_4$-alkoxy, $NR^5R^6$, and $N(R^5)_3^{\oplus}A^{\ominus}$, wherein $R^5$ and $R^6$ are independently hydrogen, unsubstituted or mono-substituted $C_1$-$C_4$-alkyl, where the substituent is amino, mono- or di-$C_1$-$C_4$-alkylamino, hydroxyl, $C_1$-$C_4$-alkoxy or $N(C_1$-$C_4$-alkyl$)_3^{\oplus}A^{\ominus}$; and $A^{\ominus}$ is a counterion selected from the group consisting of single negatively-charged ions, such as chloride, bromide, nitrate, perchlorate, benzoate, maleate, benzene sulfonate, tartrate, hemitartrate and acetate; $-CONR^5R^6$, where $R^5$ and $R^6$ are as defined above; and Het, where Het is an unsubstituted or mono- or disubstituted 5- to 7-membered mono- or bicyclic or 7- to 10- membered bicyclic heterocyclic ring, wherein the one or two heteroatoms are independently selected from the group consisting of N, O, S, NO, SO, $SO_2$ and quaternized N, and the substituent(s) is/are independently selected from the group consisting of hydroxy, thiol, $C_1$-$C_6$-alkyl, $CF_3$, $C_1$-$C_4$-alkoxy, halo, aryl, as defined above, aryl-$C_1$-$C_4$-alkyl, amino, mono- or di-$C_1$-$C_4$-alkylamino, amino-$C_1$-$C_4$-alkyl, hydroxy-$C_1$-$C_4$-alkyl, di-$C_1$-$C_4$-alkylamino-$C_1$-$C_4$-alkyl, guanidyl, guanidyl-$C_1$-$C_4$-alkyl, $CO_2H$, $CO_2$-$C_2$-$C_4$-alkyl, $NR^5R^6$ and $N(R^5)_3^{\oplus}A^{\ominus}$, wherein $R^5$, $R^6$, and $A^{\ominus}$ are as defined above;

or alternatively when the heteroatom N is present, the substituents are $-(CH_2)_q-$ or $-(CH_2)_2$-O-$(CH_2)_2$-and form a quaternary spirocyclic ring with the N-atom, wherein q is 3-to-6;

except that where X is -O-, only one of $R^{2a}$ or $R^{2b}$ is present;

$R^{2c}SO_2$-,

where

$R^{2c}$ is unsubstituted, mono- or di-substituted $C_1$-$C_4$ alkyl, wherein the substituent(s) is/are independently chosen from the group consisting of amino, mono or di-$C_1$-$C_4$-alkylamino, t-butoxycarbonylamino, carbobenzyloxyamino, acetylamino, trimethylacetylamino, hydroxy, $-SO_3H$, $-CO_2H$, $-CO_2$-$C_1$-$C_4$-alkyl, $C_1$-$C_4$-alkyl-$SO_2$- and Het, as defined above; or

Het-CO, where Het is as defined above;

$A^1$ is hydrogen or $C_1$-$C_4$-alkyl;

r is 1-to-4;

$R^3$ is hydrogen, $C_1$-$C_4$-alkyl, aryl, as defined above, aryl-$C_1$-$C_4$-alkyl, or indolyl;

$R^1$ is hydrogen, aryl, as defined above;
Het-$SO_t$-$CH_2$, wherein t is 0-to-2; or unsubstituted or monosubstituted $C_1$-$C_4$-alkyl or $C_1$-$C_4$-alkylthio-$C_1$-$C_4$-alkyl, where the substituent is on the terminal carbon atom and is chosen from the group consisting of $C_1$-$C_4$-alkoxy, amino, mono- or di- $C_1$-$C_4$-alkylamino, carboxy, $CO_2$-$C_1$-$C_4$ alkyl, aryl, as defined above, Het, as defined above, or -$CONR^5R^6$, wherein $R^5$ and $R^6$ are as defined above;

$R^7$ is $C_3$-$C_6$-alkyl;
aryl, as defined above; or
unsubstituted or mono-, di- or trisubstituted $C_3$-$C_7$-cycloalkyl, where the substituent(s) is/are independently selected from the group consisting of $C_1$-$C_4$-alkyl, trifluoromethyl, hydroxy, $C_1$-$C_4$-alkoxy and halo;

Q is 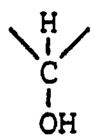 ; 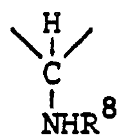 ; 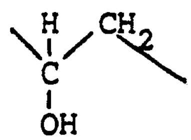 ; or 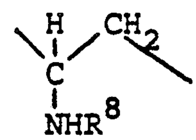 ,

where
$R^8$ is hydrogen; $C_1$-$C_4$-alkyl; formyl; $C_1$-$C_4$-alkanoyl; aroyl; $C_1$-$C_4$-alkoxycarbonyl; aryloxycarbonyl; or aryl-$C_1$-$C_4$-alkoxycarbonyl, wherein aryl is as defined above;

$R^4$ is hydrogen;

$C_2$-$C_8$-alkenyl;
mono- or disubstitued $C_2$-$C_8$-alkyl, where the substituent(s) is/are on the final 1 or/and 2 carbon atoms of the alkyl chain and is/are independently selected from the group consisting of hydroxy, carboxy, $CO_2$-$C_1$-$C_4$-alkyl, amino, mono-, di- or tri-$C_1$-$C_4$-alkyl-amino, guanidyl, $NR^5R^6$ and $N(R^5)_3^{\oplus}A^{\ominus}$, wherein $R^5$, $R^6$ and $A^{\ominus}$ are as defined above; aryl, as defined above; or

unsubstituted, mono- , di- or trisubstituted $C_3$-$C_7$-cycloalkyl, where the substituent(s) is/are independently selected from the group consisting of $C_1$-$C_4$-alkyl, trifluoromethyl, hydroxy, $C_1$-$C_4$-alkoxy and halo;

$R^9$ is -$(CH_2)_s$-$NR^{11}R^{12}$,
where
$R^{11}$ and $R^{12}$ are independently hydrogen, Het, as defined above, aryl, as defined above, $C_3$-$C_7$-cycloalkyl, or unsubstituted or monosubstituted $C_1$-$C_4$-alkyl, wherein the substituent is on the terminal carbon atom and is chosen from the group consisting of aryl, as defined above; Het, as defined above; amino; hydroxyl; mono- or di-$C_1$-$C_4$-alkylamino; $CO_2H$; $CONR^5R^6$, where $R^5$ and $R^6$ are as defined above; and $SO_3H$; and s is 1 or 2;

-$(CH_2)_tNR^{11a}R^{12a}$,
where
$R^{11a}$ and $R^{12a}$ are independently Het, as defined above, aryl, as defined above, $C_3$-$C_7$-cycloalkyl or monosubstituted $C_1$-$C_4$-alkyl, wherein the substituent is on the terminal carbon atom and is chosen from the group consisting of aryl, as defined above; Het, as defined above; amino; hydroxyl; mono- or di-$C_1$-$C_4$-alkylamino; $CO_2H$; $CONR^5R^6$, where $R^5$ and $R^6$ are as defined above; and $SO_3H$; and t is 3 or 4;

-$(CH_2)_uN(R^{11}R^{12}R^{13})^{\oplus}A^{\ominus}$,
where
u is 2-to-5; $R^{13}$ is independently chosen from the definitions of $R^{11}$; and $R^{11}$ and $A^{\ominus}$ are as defined above;

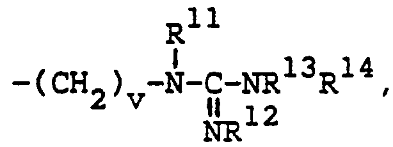

where
$R^{14}$ is independently chosen from the definitions of $R^{11}$, or together with $R^{13}$ is $-(CH_2)_4-$ or $-(CH_2)_5-$; v is 1-to-5; and $R^{11}$, $R^{12}$ and $R^{14}$ are as defined above;

$(CH_2)_s-R^{18}$,
where
$R^{18}$ is $-CH(CH_2CH_2)_2NR^{10}$,

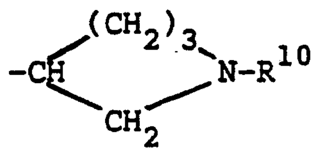

or

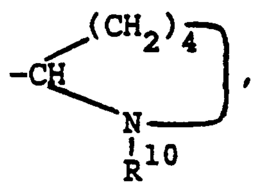

wherein $R^{10}$ is H or $C_1$-$C_4$-alkyl; and s is as defined above; or

when either $R^{10}$ or $R^{10a}$ is $C_1$-$C_4$-alkyl, also alternatively $-(CH_2)_4-NR^{11}R^{12}$, wherein $R^{11}$ and $R^{12}$ are as defined above;

$R^{10a}$ and $R^{10b}$ are independently H or $C_1$-$C_4$-alkyl;

J is

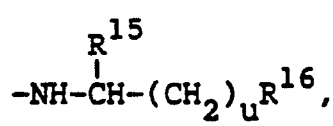

where $R^{15}$ is hydrogen, $C_1$-$C_4$-alkyl, or aryl-$C_1$-$C_4$-alkyl, wherein aryl is as defined above; $R^{16}$ is hydrogen;-$CO_2R^{17}$, wherein $R^{17}$ is hydrogen, $C_1$-$C_4$-alkyl, phenyl-$C_1$-$C_4$-alkyl or $C_5$-$C_{10}$-cycloalkyl; $CONR^{17}R^{18}$, wherein $R^{18}$ is independently selected from the definitions of $R^{17}$ and $R^{17}$ is as defined above; or, when u is not equal to 0, is also alternatively amino or mono- or di-$C_1$-$C_4$-alkylamino; and u is 0-to-4; or

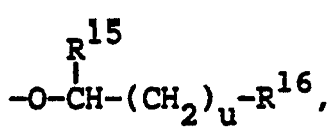

where $R^{15}$, $R^{16}$ and u are as defined above;
and pharmaceutically-acceptable salts thereof.

In the tetrapeptides of the present invention, the amino acid, A and J components have asymmetric centers and occur as racemates, racemic mixtures and as individual diastereomers, with all isomeric forms

generally being included in the present invention. In particular, asymmetric carbon atoms in the dipeptidal analogous 10, 11 position substituent have either an S or an R configuration, and all other asymmetric carbon atoms have an S configuration, with preferred chiralities indicated in the following further description.

When any variable (e.g., aryl, Het, $R^5$, $R^6$, $R^{11}$, $R^{12}$, $R^{13}$, $R^{14}$, $A^{\ominus}$, etc.) occurs more than one time in any constituent or in formula I, its definition on each ocurrence is independent of its definition at every other occurrence.

As used herein, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; and "$C_3$-$C_7$-cycloalkyl" is intended to include saturated ring groups, such as cyclopropyl, cyclobutyl, cyclopentyl, cyclohexyl (Cyh) and cycloheptyl. "Alkanoyl" is intended to include those alkylcarbonyl groups of specified number of carbon atoms, which are exemplified by formyl, acetyl, propanoyl and butanoyl; "alkenyl" is intended to include hydrocarbon chains of either a straight- or branched- configuration and one unsaturation which may occur at any point along the chain, such as ethenyl, propenyl, butenyl, pentenyl, and the like, and includes E and Z forms, where applicable; and "arylalkyl" represents aryl groups as herein defined which are attached through a straight- or branched- chain alkyl group of specified number of carbon atoms, such as, for example, benzyl, phenethyl, 3,3-diphenylpropyl, 3-indolymethyl, and the like. "Halo", as used herein, means fluoro, chloro, bromo and iodo, and "counterion" is used to represent a small, single negatively-charged specie, such as chloride, bromide, acetate, perchlorate, benzoate, maleate, benzene sulfonate, tartrate, hemitartrate and the like.

As used herein, with exceptions as noted, "aryl" is intended to mean phenyl (Ph) or naphthyl, which is optionally-substituted by from one- to three- members independently selected from the group consisting of amino (Am), mono- or di- $C_1$-$C_4$-alkyl amino, amino-$C_1$-$C_4$-alkyl, hydroxy-$C_1$-$C_4$-alkyl, di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2R^7$, $NR^5R^6$, wherein $R^7$, $R^5$ and $R^6$ are as defined above, or $N(R^5)_3^{\oplus}A^{\ominus}$ wherein $R^5$ is as defined above and $A^{\ominus}$ is a counterion, as defined herein. "Aroyl" is intended to include those aryl-carbonyl groups which are exemplified by benzoyl and naphthoyl.

The term "Het", as used herein, represents a 5- to 7-membered mono- or bicyclic or 7- to 10-membered bicyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. Fully-saturated heterocyclic substituents are preferred and those in which nitrogen is the heteroatom are also preferred, with those containing a single nitrogen atom being particularly preferred. In the case of a heterocyclic ring containing one or more nitrogen atoms, the point of attachment may be at one of the nitrogen atoms, or at any carbon atom. Examples of such heterocyclic elements include piperidyl, piperidinyl, piperazinyl, 2-oxopiperazinyl, pyrryl, pyrrolinyl, 4-piperidonyl, pyrrolidinyl, pyrazolyl, pyrazolinyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, benzothiazolyl, benzoxazolyl, furyl, thienyl, benzothienyl, thiamorpholinyl, thiamorpholinyl sulfoxide, and thiamorpholinyl sulfone. The heterocyclic moiety is further optionally-substituted by from one- to four- members independently selected from the group consisting of hydroxyl, thiol, $C_1$-$C_6$-alkyl, $CF_3$, $C_1$-$C_4$-alkoxy, halo, aryl, aryl-$C_1$-$C_4$-alkyl, amino, mono- or di-$C_{1-4}$-alkylamino, amino-$C_1$-$C_4$-alkylamino, amino-$C_1$-$C_4$-alkyl, hydroxy-$C_1$-$C_4$-alkyl, $C_1$-$C_4$-dialkylamino-$C_1$-$C_4$-alkyl, guanidyl, guanidyl $C_1$-$C_4$-alkyl, CHO, $CO_2H$, $CO_2R^7$, $NR^5R^6$, wherein $R^7$, $R^5$ and $R^6$ are as defined above, or $N(R^5)_3^{\oplus}A^{\ominus}$, wherein $R^5$ is as defined above and $A^{\ominus}$ is a counterion, as defined herein, or a spiro quaternary N bicyclic may be formed.

The following additional abbreviations have also been used herein:

| Abbreviated Designation | Amino Acid/Residue |
|---|---|
| ACHPA | (3S,4S)-4-amino-5-cyclohexyl-3-hydroxypentanoic acid |
| Arg | D- or L-arginine |
| Cal (Cha) | 3-cyclohexylalanine |
| Dab | D- or L- (2,4-diamino)butanoic acid |
| Dap | D- or L-(2,3-diamino)propionic acid |
| Gly | glycine |
| His | D- or L-histidine |
| HArg | D- or L-homologated arginine |
| HPhe | D- or L-homologated phenylalanine (homophenylalanine) |
| HLys | D- or L-homolysine |
| Ile | D- or L-isoleucine |
| Leu | D- or L-leucine |
| Lys | D- or L-lysine |
| Nle | D- or L-norleucine |
| Nva | D- or L-norvaline |
| Orn | D- or L-ornithine |
| Phe | D- or L-phenylalanine |
| Sta | statine, (3S,4S)-4-amino-3-hydroxy-6-methylheptanoic acid |
| | **Protecting Group** |
| BOC (Boc) | t-butyloxycarbonyl |
| CBZ (Cbz or Z) | benzyloxycarbonyl(carbobenzoxy) |
| DNP | 2,4-dinitrophenyl |
| EtOC | ethoxycarbonyl |

| | |
|---|---|
| OEt | ethoxy, except when it immediately follows an amino acid residue abbreviation and it represents ethyl ester |
| TBDMS | t-butyldimethylsilyl |
| | **Activating Group** |
| HOBt(HBT) | N-hydroxybenzotriazole hydrate |
| | **Condensing Agent** |
| DCC | N,N'-dicyclohexylcarbodiimide |
| | **Reagent** |
| i-$Pr_2NEt$ | diisopropylethylamine |
| TEA($Et_3N$) | triethylamine |
| TFA | trifluoroacetic acid |
| | **Coupling Reagents** |
| BOP reagent | benzotriazol-1-yloxytris(dimethyl-amino)phosphonium hexafluoro-phosphate |
| BOP-Cl | bis(2-oxo-3-oxazolidinyl)phosphinic chloride |
| | **Solvent** |
| AcOH (HOAc) | acetic acid |
| $CH_2Cl_2$ | methylene chloride |
| DMF | N,N-dimethylformamide |
| EtOAc | ethyl acetate |
| THF | tetrahydrofuran |

The novel renin inhibitory peptides of the present invention may alternately be described in terms of common amino acid components and closely-related analogs thereof, in accordance with the following formula, analogous to formula I:

A-B-D-G-T-J (II),

wherein

A is Boc, EtOC, isopropylsulfonyl, (4-carboxy)pentanoyl, (2-N-Boc-amino)ethyloxycarbonyl, or (morpholin-4-yl)carbonyl;

B is Phe, HPhe, Trp or Tyr(OMe);

D is His, Ala, Ile, Leu, Met, Nle, Nva or Phe;

G is ACHPA, Sta, AmACHPA, HomoACHPA, and analogues;

T is Arg, HArg, Orn, Dap, Dab, ($N^{\alpha}$-methyl)Lys or HLyS; and
J is n-butyl, isobutyl or benzyl.

In terms of substrate analogy, a unique aspect and essential feature of the present invention is the substitution of the G-component for the double amino acid sequence, $Leu^{10}$-$Leu^{11}$ in the endogenous pig renin substrate, or $Leu^{10}$-$Val^{11}$ in the endogenous human renin substrate

```
 7    8    9    10   11   12   13
(Pro  Phe  His  Leu  Val  Ile  His) ,
```

which substitution for both amino acids at the cleavage site rather than just one is believed to result in an improved substrate analogy and better fit to the renin enzyme in linear extent.

In addition to an increase in metabolic stability achieved with the substitution of statine analogs/residue (particularly ACHPA), and in the shortening of the peptide chain, the novel peptides of the instant invention successfully use hydrophilic and/or hydrophobic groups strategically and comprise simplified N- and C-terminii, preferably with the elimination of $\alpha$-amino acids following ACHPA at the C-terminus of these peptides. These peptides are of lower molecular weight and different structure, with an enhanced potency and duration of action, than known peptides of this class.

It will be understood that closely-related analogs of the above common amino acids, for example, aliphatic amino acids in addition to Ala, Val, Leu, and Ile, such as $\alpha$-aminobutyric acid (Abu), and substituted phenyl derivatives of Phe, are included in the broad description of the novel inhibitory peptides of the present invention represented by Formulae I and II and related definitions.

Representative preferred renin-inhibitory peptides of the present invention include the following:

Boc-Phe-His-ACHPA-HLys-NH-i-Bu
Boc-Phe-His-ACHPA-NHCH[$CH_2N(CH_3)_2$]CO-$NHCH_2CH_2NH_2$
Boc-Phe-His-ACHPA-[$N^{\epsilon}$-(pyridin-4-yl)]Lys-NH-2(S)-methylbutyl
Boc-Phe-His-ACHPA-NHCH($CH_2CH_2$NH-i-Pr)CO-$NHCH_2CH_2OH$
Boc-Phe-His-ACHPA-($N^{\epsilon}$-$CH_2CO_2H$)Lys-$NHCH_2Ph$
Boc-Phe-His-ACHPA-NH($CH_2NH_2$)CO-NHCH(2-butyl)$CH_2OH$
[Boc-Phe-His-ACHPA-($N^{\epsilon}$,$N^{\epsilon}$,$N^{\epsilon}$-trimethyl)Lys-$NHCH_2CH_2CO_2H$]$^{\oplus}Cl^{\ominus}$
Boc-Phe-His-ACHPA-($N^{\epsilon}$-benzyl)Lys-$NHCH_2CH_2CH_2OH$
Boc-Phe-His-ACHPA-($N^{\epsilon}$-benzyl)Lys-$NHCH_2CH_2CH_2CO_2H$
Boc-Phe-His-ACHPA-($N^{\epsilon}$-benzyl)Lys-$NHCH_2CH_2CH_2CO_2Et$
Boc-Phe-His-ACHPA-($N^{\epsilon}$-benzyl)Lys-$NHCH_2CH_2CH_2CONEt_2$
Boc-Phe-His-ACHPA-[$N^{\epsilon}$-(piperidin-4-yl)]Lys-$NHCH_2Ph$
Boc-(p-OMe)Phe-His-ACHPA-HLys-NH-CH(2-butyl)$CH_2NH_2$
Boc-$NHCH_2CH_2OCO$-Phe-His-ACHPA-HLys-NHCH(2-butyl)-$CH_2N(CH_3)_2$ $(CH_3)_3CCH_2CO$-$NHCH_2CH_2OCO$-Phe-His-ACHPA-HLys-$NHCH_2CH_2CH_2NEt_2$
$(CH_3)_3CCH_2CO$-$NHCH_2CH_2OCO$-Phe-His-ACHPA-($N^{\alpha}$-methyl)Lys-$NHCH_2CH_2NH_2$
Boc-Phe-His-ACHPA-NHCH[$(CH_2)_4NHC(=NCH_3)NHCH_3$]CO-$NHCH_2Ph$
Boc-Phe-His-ACHPA-($N^{\epsilon}$-imidazolin-2-yl)Lys-NH-i-Bu
Boc-Phe-($N^{\alpha}$-Me)-His-ACHPA-($N^{\epsilon}$-benzyl)Lys-$NHCH_2CH_2CH_2OH$
Boc-Phe-($N^{\alpha}$-Me)-His-ACHPA-($N^{\epsilon}$-benzyl)Lys-$NHCH_2CH_2CH_2CO_2H$
Boc-Phe-($N^{\alpha}$-Me) His-ACHPA-($N^{\epsilon}$-benzyl)Lys-$NHCH_2CH_2CH_2$ $CO_2Et$
Boc-Phe-($N^{\alpha}$-Me)His-Cal[CH(OH)$CH_2$]Val-($N^{\epsilon}$-benzyl)Lys-$NHCH_2Ph$
Boc-Phe-($N^{\alpha}$-Me)His-Cal[CH($NH_2$)$CH_2$]Val-HLys-$NHCH_2Ph$
Boc-Phe-($N^{\alpha}$-Me)His-Cal[CH(OH)$CH_2$]Val-CH($CH_2NH_2$)CO-N($CH_3$)$CH_2Ph$
Boc-Phe-($N^{\alpha}$-Me)His-Cal[CH(OH)$CH_2$]Val-NHCH($CH_2CH_2NH_2$)CO-$NHCH_2Ph$
$HO_2C(CH_2)_4CO$-Phe-His-ACHPA-HLys-$NHCH_2Ph$
$HO_2C(CH_2)_4CO$-Phe-His-ACHPA-NHCH[$CH_2N(CH_3)_2$]CO-NH-n-Bu
$HO_2CCH_2C(CH_3)_2CO$-Phe-His-ACHPA-HLys-$NEt_2$
$O(CH_2CH_2)_2NCO$-Phe-His-ACHPA-($N^{\epsilon}$-benzyl)Lys-$NHCH_2CH_2OH$
$iPrSO_2$-Phe-His-ACHPA-($N^{\epsilon}$-benzyl)Lys-$NHCH_2CH_2CO_2H$
$(CH_3)_2N(CH_2)_3CO$-Phe-His-ACHPA-HLys-$NHCH_2Ph$
$O(CH_2CH_2)_2NCO$-($N^{\alpha}$-Me)Phe-His-ACHPA-HLys-NH-i-Bu
$CH_3N(CH_2CH_2)_2NCO$-Phe-His-ACHPA-NHCH($CH_2NH_2$)CO-$NHCH_2CH_2CO_2H$
$SO_2(CH_2CH_2)_2NCO$-Phe-His-ACHPA-NHCH($CH_2CH_2NH_2$)CO-$NHCH_2Ph$
$SO_2(CH_2CH_2)_2NCO$-($N^{\alpha}$-Me)Phe-His-ACHPA-NHCH($CH_2CH_2NH_2$)CO-$NHCH_2Ph$

$HO_2CCH_2N(CH_2CH_2)_2NCO$-Phe-His-ACHPA-HLys-$NHCH_2CH_2Ph$
$HO_2CCH_2CH_2N(CH_2CH_2)_2NCO$-Phe-His-ACHPA-HLys-$NHCH_2Ph$
$HO_2CCH_2(CH_3)NCO$-Phe-His-ACHPA-HLys-$N(CH_3)CH_2Ph$
$O(CH_2CH_2)_2NCH_2CH_2SO_2$-Phe-His-ACHPA-HLys-$N(CH_3)CH_2Ph$
Boc-Phe-His-(2S-isobutyl)ACHPA-HLys-$NHCH_2CH(CH_3)CO_2H$
Boc-Phe-His-AmACHPA-HLys-$NHCH_2CH_2CH_2CO_2H$
Boc-Phe-His-ACHPA-($N^{\alpha}$-methyl)Lys-$NHCH_2CH_2CH_2OH$
Boc-Phe-His-ACHPA-($N^{\alpha}$-methyl-$N^{\epsilon}$-benzyl)Lys-$NHCH_2CH_2$ $CH_2CO_2H$
Boc-Phe-His-ACHPA-($N^{\alpha}$-methyl)Lys-$NHCH_2CH_2CH_2CO_2Et$
$HO_2CCH_2N(CH_2CH_2)_2NCO$-Phe-His-ACHPA-($\alpha$-methyl)Lys-$NHCH_2$ $CH_2Ph$
$HO_2CCH_2CH_2N(CH_2CH_2)_2NCO$-Phe-His-ACHPA-($\alpha$-methyl)Lys-$NHCH_2Ph$
$HO_2CCH_2(CH_3)NCO$-Phe-His-ACHPA-($\alpha$-methyl)Lys-$N(CH_3)$ $CH_2Ph$;
$O(CH_2CH_2)_2NCH_2CH_2SO_2$-Phe-His-ACHPA-($\alpha$-methyl)Lys-$N(CH_3)CH_2Ph$
$O(CH_2CH_2)_2NCO$-Phe-His-ACHPA-($N^{\epsilon}$,$N^{\epsilon}$-dimethyl)Lys-$NHCH_2C_6H_5$; and
Boc-Phe-His-ACHPA-($N^{\epsilon}$-cyclohexyl)Lys-NH-iBu.

The pharmaceutically-acceptable salts of the peptides of Formula I (in the form of water- or oil-soluble or dispersible products) include the conventional non-toxic salts or the quaternary ammonium salts of these peptides, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methane sulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

Peptide renin inhibitors of Formula I may be prepared in accordance with well-known procedures for preparing peptides from their constitutent amino acids.

Structures and abbreviations for the G components of the renin-inhibitory peptides of the present invention include:

1.

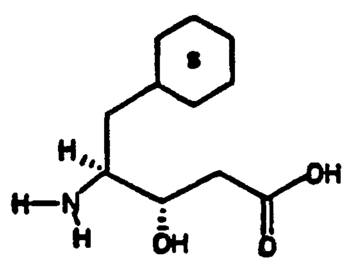

ACHPA

with B0C-ACHPA-OEt being prepared by the method described by Boger et al, J. Med. Chem 1985, 28, 1779-1790;

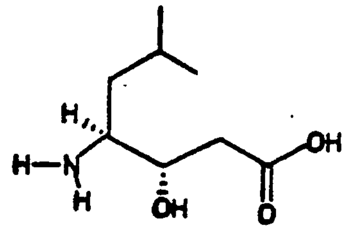

Statine (Sta)

2.

with BOC-Sta-OEt being prepared in accordance with the procedure described by Rich et al, J. Org. Chem., 43, 3624(1978);

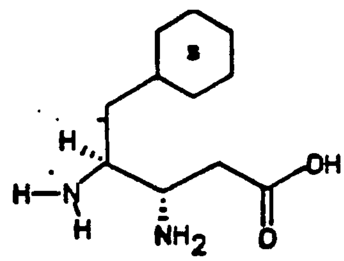

3.

AmACHPA

with BOC-AmSta(CBZ)-OH being prepared as described by Jones et al, in Peptides, Structure and Function. Proceedings of the Ninth American Peptide Symposium (eds. C. M. Deber, V.J. Hruby and K. D. Kopple) pp. 759-62, 1985, Pierce Chemical Co., Rockford, IL.; Arrowsmith et al,J. Chem. Soc. Chem. Commun. 755-7 (1986); and Raddatz et al, Published Eur. Pat. Appl. 161,588; and BOC-AmACHPA(CBZ)OH being prepared in similar fashion;

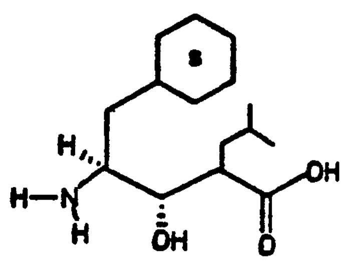

(2-isobutyl)ACHPA

4.

with efficient methods for preparing the 2-substituted statine component G in a suitably protected form being described in Sham et al, Pub. Eur. Pat Appln. 184,855 (with other pertinent references including D. Veber et al, Biochem. Soc. Trans., 12, 956-959 (1984) and H. Stein et al, Fed. Proc. 45, 869, Abstract No 4151 (1986); and

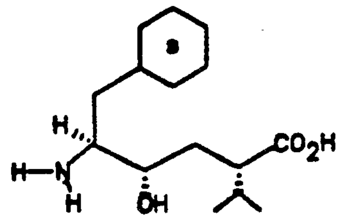

5.

Cal[CH(OH)CH$_2$]Val

with BOC-Cal[CH(OH)CH$_2$]Val-OH lactone being obtained from intermediates prepared using methods described by P. Buhlmayer et al, in Published European Patent Application 184,550-A2, and synthetic routes to similar peptide bond isosteres being described in the following:

a. Szelke et al, in Peptides, Structure and Function. Proceedings of the Eighth American Peptide Symposium (ed. V.J. Hruby and D.H. Rich) pp. 579-82, Pierce Chemical Co., Rockford, IL;

b. D.T. Pals et al in European Patent Appln. 173,481-A2,.

c. B.E. Evans et al, J. Org. Chem., 50, 4615-1625 (1985);

d. A.H. Fray et al, J.Org. Chem., 51, 4828-4833 (1986);

e. M. Szelke et al, PCT Int. Appl. WO 84 03,044; and

f. D.J. Kempf, J. Org Chem., 51, 3921-3926 (1986); and

When the unit T consists of an $N^{\alpha}$-alkyl (for example, $N^{\alpha}$-methyl) amino acid, procedures well-known in peptide synthesis are used to prepare this amino acid and to couple it. In general, the mixed anhydride procedure (with pivaloyl chloride and N-methylmorpholine) is used, as illustrated by Wenger, Helv. Chem. Acta., 1984, 67, 502-525, or the BOP-Cl procedure of Rich et al, J. Amer. Chem. Soc., 1985, 107, 4342-43, is used. $N^{\alpha}$-methyl amino acids may be prepared, for example, by the method of O'Donnell, Tetrahedron Letters, 1984, 25, 3651, while $N^{\alpha}$-alkyl amino acids generally may be prepared by the procedure of Freidinger, J. Org. Chem., 1983, 48, 77-81 or of Hansen et al, J. Org. Chem., 1985, 50, 945-950.

The peptide coupling reactions referred to below in Routes A, B, C, and D include those brought about by the action of DCC/HOBt (dicyclohexylcarbodiimide/N-hydroxybenzotriazole), DPPA (diphenylphosphorylazide), "BOP reagent" (benzotriazol-1-yloxy-tris(dimethylamino)phosphonium hexafluorophosphate), BOP-Cl (bis(2-oxo-3-oxazolidinyl)phosphinic chloride), DSO (N,N′-disuccinimidyl oxalate), and other well-known coupling reagents.

Route A:

Step A1:

BOC-AA$^3$ (1) is coupled with an amine component $R^{2c}R^{2d}NH$ which corresponds to the J element of formula I above, providing amide 2. Amide 2 is treated with anhydrous TFA, resulting in amide 3. Additional reactive functional groups in the amino component $R^{2c}R^{2d}NH$ are protected during the above steps (and those to follow below) with protecting groups, such as CBZ for amino groups, benzyl ethers for alcohols, and benzyl esters for carboxylic acids.

Step A2:

BOC-ACHPA-OEt (4) is treated with anhydrous TFA to remove the BOC protecting group, giving ACHPA-OEt (5).

Step A3:

Using standard methods, a dipeptide protected at the N-terminus with a BOC group (BOC-$AA^2$-$AA^1$) is coupled to 5, giving a coupled product 8. Alternatively, BOC-$AA^1$ is coupled to ACHPA-OEt giving 6. The coupled product 6 is treated with anhydrous TFA to removed the Boc protecting group, and the resulting product 7 is coupled with BOC-$AA^2$, giving 8.

Step A4:

The resulting coupled product 8 is treated with methanolic hydrazine, giving the corresponding hydrazide 9.

Step A5:

Hydrazide 9 is treated with acidic isoamyl nitrite, producing the corresponding acyl azide, which is treated in situ with 3, giving coupled product 10. Alternatively, coupled product 8 is treated with sodium hydroxide in THF-$H_2O$ and the resulting carboxylic acid derivative coupled with 3, giving 10.

Step A6:

The N-terminal BOC protecting group is then removed from 10 by treatment with anhydrous TFA, and the resulting tripeptide analog 11 treated with an acylating agent represented by $R^{2a}(R^{2b}))$X-CO-W, where this acylating agent is an acid chloride, another activated carboxylic acid derivative or a carbamoyl chloride, sulfonyl chloride or chloroformate. This gives acylated tripeptide derivative 12.

Step A6a:

Alternatively, compound 12 may be prepared using the following sequence: An acylated amino acid $R^{2a}(R^{2b})$X-CO-$AA^2$ is coupled to compound 7, which is prepared as described in steps A2 and A3. The resulting coupled product 8a is then treated as described for 8 in steps A3 and A4, giving first hydrazide 9a, then after coupling to $R^{2c}R^{2d}$NH, the coupled product 12.

Step A7:

During the steps above, reactive functional groups present in the side-chains of amino acid components or in the $R^{2a}R^{2b}$NH- element are protected with protecting groups. These may be CBZ groups for amines, benzyl ethers for alcohols, and benzyl esters for carboxylic acids. In the case of histidine, the BOC protecting may be used for protection of the side-chain imidazole ring; this group is removed in step A4 during the treatment with hydrazine, or alternatively in step A5 during treatment with sodium hydroxide. Thereafter the histidine imidazole ring may be left unprotected.

Step A8:

In cases where a quaternized amino group is to be introduced into the C-terminal $R^{2c}R^{2d}$NH-element of compound 13, one of the following procedures is followed to introduce the quaternized amine group:

Procedure 1:

A tertiary amine within the $R_1$ or $R_2$ group of $R^{2c}R^{2d}$NH is quaternized by treatment of $R^{2c}R^{2d}$NH-BOC with an alkyl halide and $KHCO_3$ in methanol or ethanol, or with an alkyl halide in DMF. The resulting quaternary ammonium salt is then treated with anhydrous TFA to remove the BOC protecting group, and the resulting amine used for preparation of 3 as described above. Likewise an amino acid $AA^3$, the side-chain of which contains a quaternary ammonium group, may be used for the preparation of 3.

Procedure 2:

A compound 12, in which the $R^{2c}R^{2d}N$- element or the side-chain of $AA^3$ contains a teriary amine, is prepared and then the tertiary amine is quaternized by treatment with an alkyl halide in DMF. In this latter case, histidine, when present as the $AA^1$ element, must first be reprotected as the BOC derivative by treatment of 12 with di-t-butyldicarbonate. The BOC group is then removed after step A9 by treatment of 13 with $K_2CO_3$ in methanol or with anhydrous ammonia in DMF or methanol.

Step A9:

Protecting groups are removed from 12 by hydrogenolysis, giving 13.

| Compound | Formula |
|---|---|
| 1 | BOC-$AA^3$ |
| 2 | BOC-$AA^3$-$NR^{2c}R^{2d}$ |
| 3 | $AA^3$-$NR^{2c}R^{2d}$ |
| 4 | BOC-ACHPA-OEt |
| 5 | ACHPA-OEt |
| 6 | BOC-$AA^1$-ACHPA-OEt |
| 7 | $AA^1$-ACHPA-OEt |
| 8 | BOC-$AA^2$-$AA^1$-ACHPA-OEt |
| 8a | $R^2(R^{2b})X$-CO-$AA^2$-$AA^1$-ACHPA-OEt |
| 9 | BOC-$AA^2$-$AA^1$-ACHPA-$NHNH_2$ |
| 9a | $R^{2a}(R^{2b})X$-CO-$AA^2$-$AA^1$-ACHPA-$NHNH_2$ |
| 10 | BOC-$AA^2$-$AA^1$-ACHPA-$AA^3$-$NR^{2c}R^{2d}$ |

11 $AA^2$-$AA^1$-ACHPA-$AA^3$-$NR^{2c}R^{2d}$

12(13) $R^{2a}(R^{2b})X$-CO-$AA^2$-$AA^1$-ACHPA-$AA^3$-$NR^{2c}R^{2d}$

Route B:

Step B1:

BOC-ACHPA-OEt is treated with sodium hydroxide in THF-$H_2O$, giving BOC-ACHPA.

Step B2:

BOC-ACHPA is coupled with 3, giving coupled product 14.

Step B3:

Compound 14 is treated with anhydrous TFA to remove the BOC protecting group, giving 15.

Step B4:

Compound 15 is coupled with a dipeptide protected at the N-terminus with a BOC protecting group (BOC-$AA^2$-$AA^1$), giving coupled product 17. Alternatively, 15 is coupled with BOC-$AA^1$, giving 16. Treatment of 16 with anhydrous TFA and coupling of the resulting product with BOC-$AA^2$ gives 17.

Step B5:

The N-terminal BOC protecting group is then removed from 17 by treatment with anhydrous TFA, and the resulting tripeptide analog 18 treated with an acylating agent represented by $R^{2a}(R^{2b})$X-CO-W, where this acylating agent is an acid chloride, another activated carboxylic acid derivative or a carbamoyl chloride, sulfonyl chloride or chloroformate. This gives acylated tripeptide derivative 12.

Step B6:

In cases where a quaternized amino group is to be introduced into the C-terminal $R^{2c}R^{2d}$N-element of compound 13, one of the following procedures is followed to introduce the quaternized amine group:

Procedure 1:

A tertiary amine within the $R_1$ or $R_2$ group of $R^{2c}R^{2d}$NH is quaternized by treatment of $R^{2c}R^{2d}$N-BOC with an alkyl halide and $KHCO_3$ in methanol or ethanol, or with an alkyl halide in DMF. The resulting quaternary ammonium salt is then treated with anhydrous TFA to remove the BOC protecting group, and the resulting amine used for the preparation of 3 as described in route A.

Procedure 2:

A compound 12, in which $AA^3$ or the $R^{2c}R^{2d}$N- element contains a teriary amine, is prepared and the tertiary amine is then quaternized by treatment with an alkyl halide DMF. In this latter case, histidine, when present as the $AA^1$ element, must first be reprotected as the BOC derivative by treatment of 12 with di-t-butyldicarbonate. The BOC group is then removed after step B7 by treatment of 13 with $K_2CO_3$ in methanol or with anhydrous ammonia in DMF or methanol.

Step B7:

As described above in route A, reactive functional groups in amino acid side chains of $AA^1$, $AA^2$, and $AA^3$, and in the $R^{2c}R^{2d}$N- component are protected during the coupling steps above. The protecting groups are now removed from coupled product 12 by hydrogenolysis giving compound 13.

| Compound | Formula |
|---|---|
| 14 | BOC-ACHPA-$AA^3$-$NR^{2c}R^{2d}$ |
| 15 | ACHPA-$AA^3$-$NR^{2c}R^{2d}$ |
| 16 | BOC-$AA^2$-ACHPA-$AA^3$-$NR^{2c}R^{2d}$ |
| 17 | BOC-$AA^2$-$AA^1$-ACHPA-$AA^3$-$NR^{2c}R^{2d}$ |
| 18 | $AA^2$-$AA^1$-ACHPA-$AA^3$-$NR^{2c}R^{2d}$ |

Peptides of Formula I which contain statine are prepared as described above in routes A and B except that BOC-ACHPA-OEt is replaced with BOC-Sta-OEt. In this way, peptides such as 19 may be prepared.

| | |
|---|---|
| 19 | $R^{2a}(R^{2b})$X-CO-$AA^2$-$AA^1$-Sta-$AA^3$-$NR^{2c}R^{2d}$ |

Peptides of Formula I which contain amino-ACHPA (AmACHPA) or aminostatine (AmSta) are prepared as described above in Routes A and B except that BOC-ACHPA-OEt is replaced with BOC-AmACHPA-(CBZ)-OEt or with BOC-AmSta(CBZ)-OEt. The CBZ group of the AmACHPA or AmSta element is removed by hydrogenolysis step A9 or B7. In this way, peptide such as 20 (and the corresponding AmSta analogs) may be prepared.

| | |
|---|---|
| 20 | $R^{2a}(R^{2b})X\text{-}CO\text{-}AA^2\text{-}AA^1\text{-}AmACHPA\text{-}AA^3\text{-}NR^{2c}R^{2d}$ |

Peptides of Formula I which contain a 2-substituted statine or ACHPA element as a peptide bond mimic may be prepared as described above in Routes A and B except that BOC-ACHPA-OEt is replaced with a suitably protected 2-substituted ACHPA or 2-substituted statine derivative, for example BOC-(2-allyl)-ACHPA-OEt or BOC-(2-isobutyl)Sta-OEt. In this way, peptides such as 21 may be prepared.

21 $R^{2a}(R^{2b})X\text{-}CO\text{-}AA^2\text{-}AA^1\text{-}(2\text{-}isobutyl)ACHPA\text{-}AA^3\text{-}NR^{2c}R^{2d}$

Peptides of Formula I which contain Cal[CH(OH)$CH_2$]Val as a peptide bond mimic are prepared as described below in Routes C and D.

Route C:

Step C1:

The Boc protecting group is removed from lactone 22 by treatment with anhydrous TFA, giving lactone 23.

Step C2:

Lactone 23 is coupled with a Boc-protected amino acid (Boc-$AA^1$) giving 24 or with a N-terminal Boc-protected dipeptide (Boc-$AA^2$-$AA^1$) giving 25. Compound 25 may also be prepared by treating 24 with anhydrous TFA, and coupling the product with Boc-$AA^2$. Treatment of 24 (or 25) with anhydrous TFA, and coupling of the product with $R^{2a}(R^{2b})X$-CO-W, as defined above, yields 26 (or 27).

Step C3:

Lactone 26 is treated with aqueous potassium hydroxide to give the corresponding hydroxyacid. The hydroxyacid is treated with t-butyldimethylsilyl chloride and imidazole, then with HOAc in THF-$H_2O$, giving the protected hydroxyacid 27.

Step C4:

Compound 27 is coupled with 3, giving 28. Additional reactive functional groups in the amino acid side-chains or in amino component $R^{2c}R^{2d}NH$ are protected with protecting groups such as CBZ for amino and guanidino groups, benzyl ethers for alcohols, and benzyl esters for carboxylic acids. When histidine is used as an amino acid component, the side-chain imidazole ring may be protected during step C2 with a BOC protecting group. This protecting group is removed during treatment with TFA, and the imidazole ring left unprotected during subsequent N-terminal coupling or acylation reactions. Alternatively histidine may be protected with a DNP protecting group during step C2. This group is then removed in step C3 during the potassium hydroxide treatment.

Step C5:

The silyl protecting group is removed from 28 by treatment with tetrabutylammonium fluoride in THF-DMF, giving 29.

Step C6:

In some cases, a quaternized amino group is present in the C-terminal $R^{2c}R^{2d}N$- element of compound 30. In this case, on the following procedures is used:

Procedure 1:

A tertiary amine within $R^{2c}$ or $R^{2d}$ of $R^{2c}R^{2d}NH$ is quaternized by treatment of $R^{2c}R^{2d}N$-BOC with an alkyl halide and $KHCO_3$ in methanol or ethanol or with an alkyl halide in DMF. The resulting quaternary ammonium salt is then treated with anhydrous TFA to remove the BOC protecting group, and the resulting amine used for preparation of compound 3.

Procedure 2:

A compound 29 containing a tertiary amine in the or $R^{2c}R^{2d}N$- or $AA^3$ element is prepared. If histidine is present as the $AA^1$ component, the imidazole ring is protected next by treatment with di-t-butyldicarbonate and $Et_3N$ in methanol or DMF. The tertiary amine present in the $AA^3$ or $R^{2C}R^{2d}N$- element is then quaternized by treatment with an alkyl halide in DMF.

Step C7:

Protecting groups are removed from 29 by hydrogenolysis, giving 30.

| | |
|---|---|
| 22 | BOC-Cal[CH(OH)$CH_2$]Val lactone |
| 23 | Cal[CH(OH)$CH_2$]Val lactone |
| 24 | BOC-$AA^1$-Cal[CH(OH)$CH_2$Val lactone |
| 25 | BOC-$AA^2$-$AA^1$-Cal[CH(OH)$CH_2$]Val lactone |
| 26 | $R^{2a}$($R^{2b}$)X-CO-$AA^2$-$AA^1$-Cal[CH(OH)$CH_2$]Val lactone |
| 27 | $R^{2a}$($R^{2b}$)X-CO-$AA^2$-$AA^1$-Cal[CH(OTBDMS)$CH_2$]Val-OH |
| 28 | $R^{2a}$($R^{2b}$)X-CO-$AA^2$-$AA^1$-Cal[CH(OTBDMS)$CH_2$]Val-$AA^3$ -$NR^{2c}R^{2d}$ |
| 29 (30) | $R^{2a}$($R^{2b}$)X-CO-$AA^2$-$AA^1$-Cal[CH(OH)$CH_2$]Val-$AA^3$-$NR^{2c}R^{2d}$ |

Route D:

Step D1:

Lactone 22 is treated with potassium hydroxide to give the corresponding hydroxyacid 31. The hydroxyacid 31 is treated with t-butyldimethylsilyl chloride and imidazole, then with HOAc in THF-$H_2O$, giving the protected hydroxyacid 32.

Step D2:

Protected hydroxyacid 32 is coupled with 3, giving 33. Additional reactive functional groups in the amino component $R^{2c}R^{2d}NH$ are protected with protecting groups such as CBZ for amino and guanidino groups, benzyl ethers for alcohols, and benzyl esters for carboxylic acids.

Step D3:

The BOC protecting group is removed from 33 by treatment with anhydrous TFA, giving 34.

Step D4:

Compound 34 is coupled sequentially with BOC-protected amino acids (BOC-$AA^1$ and BOC-$AA^2$) or with a dipeptide with an N-terminal BOC protecting group (BOC-$AA^2$-$AA^1$), giving coupled product 35.

Step D5:

The N-terminal BOC protecting group of 35 (41) is removed by treatment with anhydrous TFA in $CH_2Cl_2$, giving 36.

Step D6:

Compound 36 is treated with an acylating agent represented by $R^{2a}(R^{2b})$X-CO-W, as defined above. This gives acylated tripeptide derivative 28. Additional reactive functional groups in $R^{2a}(R^{2b})$X-CO-W are protected with protecting groups as described above.

Step D7:

The silyl protecting group is removed from 28 by treatment with fluoride, giving 29.

Step D8:

In some cases a quaternized amino group is present in the C-terminal $R^{2c}R^{2d}N$- element of compound 30. In this case, one of the following procedures is followed:

Procedure 1:

A tertiary amine within $R^{2c}$ or $R^{2d}$ of $R^{2c}R^{2d}NH$ is quaternized by treatment of $R^{2c}R^{2d}N$-BOC with an alkyl halide and $KHCO_3$ in methanol or ethanol or with an alkyl halide in DMF. The resulting quaternary ammonium salt is then treated with anhydrous TFA to remove the BOC protecting group, and the resulting

amine used to prepare 3 as described in route A.

Procedure 2:

A compound 29 is prepared as described above. If histidine is present as the $AA^1$ component, the imidazole ring is protected next by treatment with di-t-butyldicarbonate and $Et_3N$ in methanol or DMF. The tertiary amine present in the $R^{2c}R^{2d}N$- element is then quaternized by treatment with an alkyl halide in DMF.

Step D9:

It is understood that during the steps above, the reactive functional groups in the side-chains of amino acid components are protected with protecting groups, which are removed in the above hydrogenolysis step. These protecting groups may be CBZ for amines and guanidines, and benzyl ethers for alcohols. If histidine is present as $AA^1$, the side-chain imidazole ring is protected with a BOC group which is removed by TFA in step D3. Thereafter the side-chain of histidine is left unprotected. Alternatively, histidine with a DNP protecting group may be used. In this case, the DNP group is removed after step D10 by treatment of 30 with thiophenol.

Step D10:

Protecting groups are removed from 29 by hydrogenolysis, giving 30.

31 BOC-Cal[$CH(OH)CH_2$]Val-OH

32 BOC-Cal[$CH(OTBDMS)CH_2$]Val-OH

33 BOC-Cal[$CH(OTBDMS)CH_2$]Val-$AA^3$-$NR^{2c}R^{2d}$

34 Cal[$CH(OTBDMS)CH_2$]Val-$AA^3$-$NR^{2c}R^2$

35 BOC-$AA^2$-$AA^1$-Cal[$CH(OTBDMS)CH_2$]Val-$AA^3$-$NR^{2c}R^{2d}$

36 $AA^2$-$AA^1$-Cal[$CH(OTBDMS)CH_2$]Val-$AA^3$-$NR^{2c}R^{2d}$

The novel peptides of the present invention possess a high degree of activity in treating renin-associated hypertension, hyperaldosteronism and/or congestive heart failure in humans, as well as in other warm-blooded animals such as mice, rats, horses, dogs and cats.

For these purposes, the peptides of the present invention may be administered orally, parenterally (including subcutaneous injections, intravenous, intramuscular, intrasternal injection or infusion techniques), by inhalation spray, or rectally, in dosage unit formulations containing conventional non-toxic, pharmaceutically-acceptable carriers, adjuvants and vehicles.

Thus, in accordance with the present invention there is further provided a method of treating and a pharmaceutical composition for treating renin-associated hypertension, hyperaldosteronism, and/or congestive heart failure. This treatment involves administering to a patient in need of such treatment a pharmaceutical composition comprising a pharmaceutical carrier, optionally with an adjuvant, and a therapeutically-effective amount of a peptide of the formula:

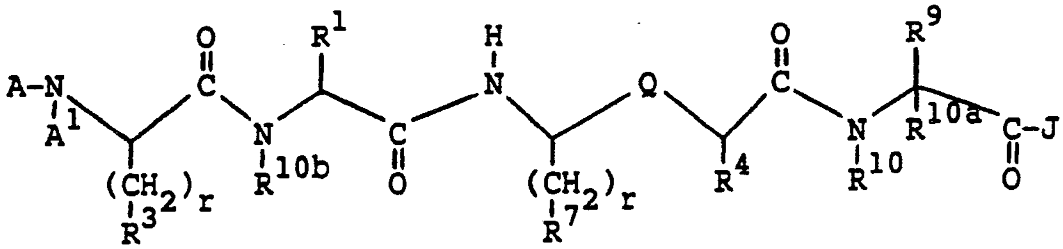

wherein A, $A^1$, $R^1$, $R^3$, $R^4$, $R^7$, $R^9$, $R^{10}$, $R^{10a}$, $R^{10b}$, J and r are defined above, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions; or suppositories.

When administered orally as a suspension, these compositions may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquify and/or dissolve in the rectal cavity to release the drug.

Dosage levels of the order of 0.02 to 2.0 grams-per-day are useful in the treatment of the above-indicated conditions, with oral doses two-to-five times higher. For example, renin-associated hypertension and hyperaldosteronism are effectively treated by the administration of from 10 to 50 milligrams of the compound per kilogram of body weight from one to three times per day. It will be understood, however, that the specific dose level and frequency of dosage for any particular patient will depend upon a variety of factors including the activity of the specific compound employed, the metabolic stability and length of action of that compound, the age, body weight, general health, sex, diet, mode and time of administration, rate of excretion, drug combination the severity of the particular condition, and the host undergoing therapy.

The present invention is also directed to combinations of the novel renin-inhibitory peptides of Formula I with one or more antihypertensive agents selected from the group consisting of diuretics, $\alpha$-and/or $\beta$-adrenergic blocking agents, CNS-acting agents, adrenergic neuron blocking agents, vasodilators, angiotensin I converting enzyme inhibitors, calcium channel blockers, and other antihypertensive agents.

For example, the compounds of this invention can be given in combination with such compounds or salt or other derivative forms thereof as:

<u>Diuretics:</u>

acetazolamide; amiloride; bendroflumethiazide; benzthiazide; bumetanide; chloro thiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

<u>$\alpha$-Adrenergic Blocking Agents:</u>

dibenamine; phentolamine; phenoxybenzamine; prazosin, tolazoline;

β-Adrenergic Blocking Agents:

atenolol; metoprolol; nadolol; propranolol; timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);
((±)-1-(4-((2-isopropoxyethoxy)methyl)phenoxy)-3-isopropylamino-2-propanol)fumarate) (bisoprolol);
(4-(2-hydroxy-3-[4-(phenoxymethyl)-piperidino]propoxy)-indole);
(carbazolyl-4-oxy-5,2-(2-methoxyphenoxy)-ethylamino-2-propanol);
(1-((1,1-dimethylethyl)amino)-3-((2 methyl-1H-indol-4-yl)oxy)-2-propanol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[(2-indol-3-yl-1,1-dimethylethyl)amino]propoxy]benzonitrile HCl) (bucindolol);
(α-[(tert.butylamino)methyl]-7-ethyl-2-benzofuranmethanol) (bufuralol);
(3-[3-acetyl-4-[3-(tert.butylamino)-2-hydroxypropyl]phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3′-acetyl-4′-(2-hydroxy-3-isopropylaminopropoxy)acetanilide HCl) (diacetolol);
(methyl-4-[2-hydroxy-3-[(1-methylethyl)aminopropoxy]]benzenepropanoate HCl) (esmolol);
(erythro-DL-1-(7-methylindan-4-yloxy)-3-isopropylaminobutan-2-ol);
(1-(tert.butylamino)-3-[O-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2-hydroxy-5-[(R)-1-hydroxy-2-[(R)-(1-methyl-3-phenylpropyl)amino]ethyl]benzamide);
(4-hydroxy-9-[2-hydroxy-3-(isopropylamino)-propoxy]-7-methyl-5H-furo[3,2-g][1]-benzopyran-5-one) (iprocrolol);
((-)-5-(tert.butylamino)-2-hydroxypropoxy]-3,4-dihydro-1-(2H)-naphthalenone HCl) (levobunolol);
(4-(2 hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropyl aminopropoxy)phenyl]ethyl-N′-isopropylurea) (pafenolol);
(3-[[(2-trifluoroacetamido)ethyl]amino] -1-phenoxypropan-2-ol);
(N-(3-(o-chlorophenoxy)-2-hydroxypropyl)-N′-(4′-chloro-2,3-dihydro-3-oxo-5-pyridazinyl)ethylenediamine);
((±)-N-[3-acetyl-4-[2 hydroxy-3-[(1-methylethyl)amino]propoxy]phenyl]butanamide) (acebutolol);
((±)-4′-[3-(tert-butylamino)-2-hydroxypropoxy]spiro[cyclohexane-1,2′-indan]-1′-one) (spirendolol);
(7-[3-[[2-hydroxy-3-[(2-methylindol-4-yl)oxy]propyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-(tert.butylamino)-2-hydroxy-propoxy)benzonitrile HCl) (bunitrolol);
((±)-2′-[3-(tert-butylamino)-2-hydroxypropoxy-5′-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbostyril HCl) (carteolol);
(1- (tert.butylamino)-3-(2,5-dichlorophenoxy)-2-propanol) (cloranolol);
(1-(inden-4(or 7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy-1-naphthyl)oxy]-2-propanol) (nadolol);
((S)-1-(2-cyclopentylphenoxy)-3-[(1,1-dimethylethyl)amino]-2-propanol sulfate (2:1)) (penbutolol),
(4′-[1-hydroxy-2-(amino)ethyl]methanesulfonanilide) (sotalol);
(2-methyl-3-[4-(2-hydroxy-3-tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-β-methylcinnamonitrile) (pacrinolol);
((±)-2-(3′-tert.butylamino-2′-hydroxypropylthio)-4-(5′-carbamoyl-2′-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);

(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);
(3,4-dihydro-8-(2-hydroxy-3-isopropylaminopropoxy)-3-nitroxy-2H-1-benzopyran) (nipradolol);

α- and β-Adrenergic Blocking Agents:

((±)-1-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol HCl);
(4-hydroxy-α-[[3-(4-methoxyphenyl)-1-methylpropyl]aminomethyl]-3-(methylsulfinyl)-benzmethanol HCl) (sulfinalol);
(5-[1-hydroxy-2-[[2-(o-methoxyphenoxy)ethyl]amino]ethyl]-2-methylbenzenesulfonamide HCl);
(5-[1-hydroxy-2-[(1-methyl-3-phenylpropyl)amino]ethyl]salicylamide HCl) (labetalol);
(1-((3-chloro-2-methyl-1H-indol-4-yl)oxy)-3-((2-phenoxyethyl)amino)-2-propanol-hydrogenmalonate) (ifendolol);
(4-(2-hydroxy-3-[(1-methyl-3-phenylpropyl)amino]propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)-3,4-dihydroxy)quinoxolin-2(1H)-one);

CNS-Acting Agents:

clonidine; methyldopa;

Adrenergic Neuron Blocking Agents:

guanethidine; reserpine and other rauwolfia alkaloids such as rescinnamine;

Vasodilators:

diazoxide; hydralazine; minoxidil;

Angiotensin I Converting Enzyme Inhibitors:

1-(3-mercapto-2-methyl-1-oxopropyl)-L-proline (captopril);
(1-(4-ethoxycarbonyl-2,4(R,R)-dimethylbutanoyl)indoline-2(S)-carboxylic acid);
(2-[2-[[1-(ethoxycarbonyl)-3-phenyl-propyl]amino]-1-oxopropyl]-1,2,3,4-tetrahydro-3-isoquinoline carboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)-cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1-(S)-carboxy-3-phenylpropyl]-L-lysyl-L-proline (lisinopril);

Calcium Channel Blockers:

α-[3-[[2-(3,4-dimethoxyphenyl)ethyl]methylamino]propyl]-3,4-dimethoxy-α-(1-methylethyl)benzeneacetonitrile (verapamil);
1,4-dihydro-2,6-dimethyl-4-(2-nitrophenyl)-3,5-pyridinedicarboxylic acid dimethyl ester (nifedipine);
2-(2,2-dicyclohexylethyl)piperidine (perhexiline);
N-(1-methyl-2-phenylethyl)- -phenylbenzenepropanamine (prenylamine);
3-(aminosulfonyl)-4-chloro-N-(2,3-dihydro-2-methyl-1H-indol-1-yl)benzamide (indapamide);
(2′-(2-diethylaminoethoxy)-3-phenylpropiophenone (etafenone);
(4-[4,4-bis-(4-fluorophenyl)butyl]-N-(2,6-dimethylphenyl)-1-piperazineacetamide) (lidoflazine);
(2-(N-benzyl-N-methylamino)ethylmethyl-2,6-dimethyl-4-(m-nitrophenyl)-1,4-dihydro-3,5-pyridinedicarboxylate HCl) (nicardipine);
(N-(3,4-dimethoxyphenethyl)-2-(3,4-dimethoxyphenyl)-N-methyl-m-dithiane-2-propylamine-1,1,3,3-tetraoxide) (tiapamil);
(5,6-dimethoxy-2-(3-[(α-(3,4-dimethoxy)phenylethyl)methylamino]propyl)phthalimidine) (falipamil);
(β-[(2-methylpropoxy)methyl]-N-phenyl-N-phenylmethyl-1-pyrrolidineethanamine HCl monohydrate) (bepridil);
((+)-cis-3-(acetyloxy)-5-[2-(dimethylamino)ethyl]-2,3-dihydro-2-(4-methoxyphenyl)-1,5-benzothiazepin-4-(5H)-one) (diltiazem);
((E)-1-[bis-(p-fluorophenyl)methyl]-4-cinnamylpiperazine di HCl) (flunarizine);
(5-[(3,4-dimethoxyphenethyl)methylamino]-2-isopropyl-2-(3,4,5-trimethoxyphenyl)valeronitrile (gallopamil);
(ethylmethyl(2,3-dichlorophenyl)-1,4-dihydro-2,6-dimethyl-3,5-pyridinedicarboxylate (felodipine);
(isopropyl-2-methoxyethyl-1,4-dihydro-2,6-dimethyl-4-(3-nitrophenyl)-3,5-pyridinecarboxylate) (nimodipine);
(3-ethyl-5-methyl-1,4-dihydro-2,6-dimethyl-4-(3-nitrophenyl)-3,5-pyridine dicarboxylate) (nitrendipine);

Other Antihypertensive Agents:

aminophylline; cryptenamine acetates and tannates; deserpidine; meremethoxylline procaine; pargyline; trimethaphan camsylate; and the like, as well as admixtures and combinations thereof.

Typically, the individual daily dosages for these combinations can range from about one-fifth of the minimally-recommended clinical dosages to the maximum recommended levels for the entities when they are given alone. Coadministration is most readily accomplished by combining the active ingredients into a suitable unit dosage form containing the proper dosages of each. Other methods of coadministration are, of course, possible.

The renin-inhibitory novel peptides of the present invention may also be utilized in *in vivo* or *in vitro* diagnostic methods for the purpose of establishing the significance of renin as a causative or contributory factor in hypertension, hyperaldosteronism or congestive heart failure in a particular patient.

In the *in vivo* method, a novel peptide of the present invention is administered to a patient, preferably by intravenous injection, although parenteral administration is also suitable, at a hypotensive dosage level in a single dose of from 0.1 to 10 mg per kg of body weight, and the resulting transitory fall in blood pressure, if it occurs, indicates supranormal plasma renin levels.

*In vitro* methods which may be employed involve incubating a body fluid, preferably plasma, with a novel peptide of the present invention according to methods described in Boger *et al.*, *J. Med. Chem.*, 1985, *28*, 1789-1790.

The following are intended to exemplify the present invention, without, however, limiting it.

## EXAMPLE 1

### Preparation of Boc-Phe-His-ACHPA-NHCH[$CH_2N(CH_3)_2$]CONH-iBu [N-t-butoxycarbonyl-L-phenylalanyl-L-histidyl-(3S,4S)-4-amino-5-cyclohexyl-3-hydroxypentanoyl-L-$N^{\beta}$,$N^{\beta}$-dimethyl-3,4-diamino-propionyl isobutylamide]

#### Step A:

To a solution of Boc-NHCH[$CH_2NH$(Cbz)]COOH-(1.82g, 5.384 mmol) and N-methylmorpholine(0.65 ml)

in dry THF(30 ml) was added isobutylchloroformate (0.698ml, 5.384 mmol) at -15 °C. After 5 minutes of stirring, isobutylamine(0.59ml) was added, and the mixture was stirred at that temperature for 2 hours and then at room temperature overnight. The reaction was diluted with ethylacetate (60 ml) and washed with water, sat.bicarbonate solution, 5% citric acid, water, dried ($MgSO_4$) and concentrated in vacuo to give white solid (1.8g). NMR($CDCl_3$): consistent with the structure. FAB mass spetral data: 394($\overline{M+H}$).

Step B: ACHPA-OEt hydrochloride

A cooled (ice-bath) solution of Boc-ACHPA-OEt (65g) in absolute ethanol (400 ml) was stirred vigorously as anhydrous HCl was bubbled in. After 10 minutes, the cooling bath was removed and the additin of HCl was continued until the temperature of the solution had reached 35 °C. At this stage, TLC showed complete disappearance of the starting material. The solution was concentrated to a thick oil, which was reconcentrated twice from toluene (2 X 100 ml) and dried in vacuo overnight (55.5g). TLC (silica gel) in $CH_2Cl_2$-MeOH-AcOH (90:10:1) Rf 0.4

Step C: Boc-Phe-His(Boc)-ACHPA-OEt

To a cooled (0 °C) solution of ACHPA-OEt hydrochloride (55.5g) in $CH_2Cl_2$ (500 ml) were added diisopropylethylamine (6 ml) and then Boc-Phe-His(Boc)-OH (96.5g), giving a clear solution. 1-Hydroxybenzotriazole hydrate (34g) and additional diisopropylethylamine (35 ml) were then added, followed by DCC (40g). The mixture was stirred (1 hour) and the ice bath was removed, with stirring being continued (18 hours). The dicyclohexylurea (DCU) formed was removed by filtration and the filtrate was concentrated to a thick slurry, which was taken up in ethylacetate (6 liters) and 0.5M citric acid solution (2 liters). The organic phase was separated and washed with citric acid solution, water, saturated $NaHCO_3$ (600ml), water (2 X 2 liters) and brine, dried ($Na_2SO_4$). The resulting solution was concentrated to a thick slurry. The pure product was obtained as a white solid (97g), after crystallization from ethylacetate. m.p. 156.7-158.8 °C. TLC (silica gel) in ethylacetate-hexane (4:1) Rf 0.35. [α]D -23.6

Step D: Boc-Phe-His-ACHPA-$NHNH_2$

410g of hydrazine hydrate was added to a solution of Boc-Phe-His(Boc)-ACHPA-OEt (97g) in methanol (1.5 liters) at 0 °C, and the reaction was allowed to warm to room temperature over a period of 1 hour, before being cooled again (ice-bath) and stirred for 18 hours. The mixture was concentrated to a thick slurry, then diluted with water (500 ml) and filtered. The solid was washed with water (3 X 500 ml) on the filter and dissolved in boiling 95% ethanol (700 ml), filtered while hot, and cooled. The resulting solid was filtered and dried to give the product (74.5g), m.p. 186.1-187.7 °C. TLC (silica gel) in $CH_2Cl_2$-MeOH (3:1). Rf 0.5 . [α]D -37.5.

Step E:

The aforementioned compound (0.786g) obtained from Step A was reacted with 50% TFA in $CH_2Cl_2$ (7 ml) at room temperature for 30 minutes. The reaction was concentrated in vacuo and the residue was dried overnight over NaOH under vaccum before using in the coupling step.

Step F:

To a cooled (-30 °C) solution of Boc-Phe-His-ACHPA-$NHNH_2$ (1.226 g, 2 mmol) in DMF (15 ml), conc. HCl (0.9 ml, 11 mmol) was added followed by isoamylnitrite (0.295 ml, 2.2 mmol). The reaction was stirred and the temperature was maintained between -18 °C and -22 °C for 3 hours. The reaction was then neutralized at that temperature with the addition of diisopropylethylamine (2.1 ml), and a solution of $NH_2CH$-[$CH_2NH$(Cbz)]NH-iBu obtained from Step E was added. The mixture was allowed to stand at 0 °C for 72 hours. The crude product was precipitated from the reaction by the addition of ice-water, extracted with ethylacetate and the organic layer was dried ($MgSO_4$) and evaporated to give a foam (1.4 g). The crude

material was finally purified by MPLC (silica gel) using $CHCl_3$-MeOH-$NH_4OH$ (100:10:1) to give the pure product as a foam (0.8 g). TLC (silica gel): Rf 0.62 in chloroform methanol-water- acetic acid (100-20-3-0.5). NMR($CD_3OD$): Consistent with the desired structure. FAB mass spectral data: 875(M + H)

Step G:

Boc-Phe-His-ACHPA-NHCH[$CH_2$NH(Cbz)]-iBu obtained in Step F (0.5 g) was dissolved in methanol (25 ml) and hydrogenated in presence of conc. HCl(0.046 g) at 40 psi over Pd-C(10%) (0.1 g) for 18 hours. Removal of the catalyst and the solvent gave the desired product as a glass like solid, which was dissolved in methanol (2 ml) and precipitated with dry ether to give amorphous solid (0.4 g). NMR ($CD_3OD$): consistent with the desired structure. FAB mass spectral data: 741(M + H).

Step H:

Boc-Phe-His-ACHPA-NHCH[$CH_2NH_2$]-iBu (0.12 g) was dissolved in dry methanol (5 ml), and treated with 5 equivalents of formalin and powdered 4Å molecular sieves. After 30 minutes of stirring, the solution was filtered through a pad of celite and a solution of $NaCNBH_3$ (3 equiv) in methanol (1 ml) was added slowly. After stirring overnight, the reaction was concentrated, acidified with AcOH and the crude product, thus obtained, was purified on LH-20 using methanol. The material was further purified by flash chromatography (silica gel) using chloroform:methanol:$NH_4OH$ (40:10:1) to give the pure product as a glass like solid (0.063 g). NMR($CD_3OD$): consistent with the desired structure. FAB mass spectral data:769(M + H).

## EXAMPLE 2

Preparation of Boc-Phe-His-ACHPA-NHCH[$CH_2$CO-N($CH_2CH_2$)NH]CO-NHiBu hydrochloride

A. Boc-NHCH[$CH_2$CO-N($CH_2CH_2$)NHCbz]CO-NHiBu was prepared according to the procedure described in step A of Example 1, by replacing Boc-NHCH[$CH_2$NH(Cbz)]COOH with Boc-NHCH[$CH_2$CON-($CH_2CH_2$)$_2$NCbz]COOH. NMR($CDCl_3$): consistent with the desired structure. FAB mass spectral data: 505-(M + H).

B. Boc-Phe-His-ACHPA-NHCH[$CH_2$CON($CH_2CH_2$)$_2$N]CO-NHiBu hydrochloride was prepared according to the procedures described in Example 1. NMR($CD_3OD$): consistent with the desired structure. FAB mass spectral data: 845(M + H).

## EXAMPLE 3

N-[(Morpholin-4-yl)carbonyl]-Phenylalanine

To a suspension of phenylalanine methyl ester hydrochloride (1gm,0.00465mol) in methylene chloride (10ml) was added triethylamine(2ml) followed by carbonyldiimidazole (0.83gm, 1 equiv.). After stirring for 1 hr at room temperature, morpholine was added (0.8 ml, 2 equiv.) and the reaction was stirred a further 2 hr until TLC (silica, EtOAc) showed the reaction to be complete. The reaction mixture was washed with HCl (1N), dried over $Na_2SO_4$, filtered and evaporated in vacuo. The product was purified by chromatography (silica gel, EtOAC) to give N-[(morpholin-4-yl)carbonyl]-phenylalanine methyl ester (1gm). TLC (silica gel, EtOAc) Rf = 0.52. NMR($CDCl_3$) 3.1(m,2H), 3.25-3.35(m,4H), 3.65-3.7 (m,4H), 3.75(s,3H), 4.75-4.85(m,1H)-,7.1(d,2H), 7.3(m,3H).

To a solution of the aforementioned material (0.485gm) in methanol (3ml) at room temperature was added a solution of KOH (1N,5.3 ml). After stirring for 3hr the reaction mixture was acidified with HCl (3N), and extracted with methylene chloride. The combined organic fractions were dried as usual, filtered and evaporated to dryness to give 0.39 gm of the title compound. NMR($CDCl_3$) 3.2(m,2H), 3.25-3.35(m,4H),

3.35-3.45(m,4H), 4.75-4.86(m,1H), 7.2(d,2H), 7.25-7.35(m,3H).

## EXAMPLE 4

N-[(4-Boc-Piperazin-1-yl)carbonyl]-phenylalanine

This compound was prepared according to the procedures outlined in Example 3 using N-Boc-piperazine, phenylalanine, and carbonyldiimidazole. NMR($CDCl_3$) 1.45(s,9H), 3.1-3.2(2d,2H), 3.3-3.4(m,4H), 3.3-3.45 (m,4H), 4.55-4.65(m,1H), 4.8(d,1H), 7.2(d,2H), 7.25-7.35(m,3H).

## EXAMPLE 5

N-[(Morpholin-4-yl)carbonyl]-Phe-His-ACHPA-Lys-$NHCH_2$-$C_6H_5$

Step A: Boc-ACHPA-Lys(Cbz)-$NHCH_2C_6H_5$

Boc-ACHPA-OH (3.1 g, 10 mmols), Lys(Cbz)-$NHCH_2C_6H_5$ hydrochloride (obtained from the treatment of 4.75 g of Boc-Lys(Cbz)-$NHCH_2C_6H_5$ with HCl in dioxane (4N) (4 ml) at room temperature for one hour) and diisopropylethylamine (6.85 ml) are dissolved in methylene chloride (60 ml), cooled (0°C), and BOP reagent (6.45 g, 14.6 mmols) is added. The mixture is stirred at that temperature 2 h and then 24 h at room temperature. The solvent is removed in vacuo and the residue is dissolved in ethylacetate (100 ml). The organic phase is washed with water, saturated $NaHCO_3$ and water, then dried ($MgSO_4$) and evaporated in vacuo to give the crude product, which is purified by medium pressure chromatography (MPLC) on silica gel to produce the pure product.

Step B: Boc-His(DNP)-ACHPA-Lys(Cbz)-$NHCH_2C_6H_5$

A solution of Boc-ACHPA-Lys(Cbz)-$NHCH_2C_6H_5$ in MeOH saturated with HCl is stirred 1 hr at room temperature whereupon it is thoroughly evaporated in vacuo. This material is combined in methylene chloride with diisopropylethylamine and Boc(N-im DNP)-histidine and benzotriazol-1-yloxytris-(dimethylamino)phosphoniumhexafluorophosphate ("BOP"). After stirring for 14 hr the reaction is evaporated in vacuo, redissolved in EtOAC, washed with HCl(1N), sat. $NaHCO_3$, dried over $Na_2SO_4$, evaporated and purified by chromatography to give the title compound.

Step C: N-[(Morpholin-4-yl)carbonyl]-Phe-His-ACHPA-Lys(Cbz)-$NHCH_2C_6H_5$

Boc-His(DNP)-ACHPA-Lys(Cbz)-$NHCH_2C_6H_5$ is deprotected with sat.HCl in MeOH and coupled with N-[(morpholin-4-yl)carbonyl]-phenylalanine in the presence of BOP reagent (benzotriazol-1-yloxy tris-(dimethylamino)phophonium hexafluorophosphate) in methylenechloride at room temperature. The coupled product is isolated, redissolved in methylenechloride and treated with thiophenol at room temperature. The crude product is purified on LH-20 using methanol, to give the title compound.

Step D: N-[(Morpholin-4-yl)carbonyl]-Phe-His-ACHPA-Lys-$NHCH_2C_6H_5$

The compound from Step C in methanol is added to a solution of HBr(30%) in AcOH at 0°C and the mixture is stirred at that temperature for 15 minutes and at room temperature for an additional 20 minutes. The reaction is evaporated to dryness in vacuo and the residue is dissolved in methanol and treated with propylene oxide. The resulting precipitate is filtered, washed and dried in vacuo to give the title compound.

## EXAMPLE 6

N-[(Morpholin-4-yl)carbonyl]-Phe-His-ACHPA-($N^{\epsilon}$,$N^{\epsilon}$-dimethyl)Lys-$NHCH_2C_6H_5$

The compound obtained in Example 5 is converted into the title compound according to the procedure in Step H of Example 1.

## EXAMPLE 7

Preparation of Boc-Phe-His-ACHPA-HArg-NH-iBu [N-t-butoxycarbonyl-L-phenylalanyl-L-histidyl-(3S,4S)-4-amino-5-cyclohexyl-3-hydroxypentanoyl-L-homoarginyl isobutylamide]

Step A: Boc-Phe-His-ACHPA-Lys(Cbz)-NHiBu

The titled compound was prepared according to the procedure described in Example 1 (Steps A-F), and was obtained as a glass like solid. NMR($CD_3OD$): consistent with the desired structure. FAB mass spectral data: 917(M + H).

Step B: Boc-Phe-His(Boc)-ACHPA-Lys(Cbz)-NH-iBu

The compound in step A is treated with $Boc_2O$ (1.5 equiv.) in DMF at room temperature to give the title compound after isolation. This compound is directly used in next step without further purification.

Step C: Boc-Phe-His-ACHPA-HArg-NH-iBu

A methanolic solution of Boc-Phe-His(Boc)-ACHPA-Lys(Cbz)-NHiBu is hydrogenated over Pd-C(10%) at 40 psi for 4 hours. The catalyst is removed by filtration and the filtrate upon concentration in vacuo gives Boc-Phe-His(Boc)-ACHPA-Lys-NHiBu. The product obtained above is dissolved in methanol and is treated with 1-guanyl-3,5-dimethylpyrazole [prepared according to the procedure described in Bannard et al., Canadian J. Chem., 36, 1541 (1958)] and diisopropylethylamine. The mixture is refluxed overnight and then evaporated in vacuo. The residue is triturated with ether, filtered and purified on LH-20 using methanol. The product is then treated with saturated $NH_3$ in methanol to give the title compound.

## EXAMPLE 8

Preparation of Boc-Phe-His-ACHPA-NHCH[$CH_2NHC(=NH)NH_2$]-NHiBu [N-t-butoxycarbonyl-L-phenylalanyl-L-histidyl-(3S,4S)-4-amino-5-cyclohexyl-3-hydroxypentanoyl-L-$N^{\beta}$-formamido-2,3-diaminopropionyl isobutylamide]

The title compound is prepared from Boc-Phe-His-ACHPA-NHCH[$CH_2NH$(Cbz)]CONH-iBu (obtained in Example 1) according to the procedure described in Example 7.

## EXAMPLE 9

Preparation of Boc-Phe-His-ACHPA-($N^{\epsilon}$-cyclohexyl)Lys-NH-iBu [N-t-butoxycarbonyl-L-phenylalanyl-L-histidyl-(3S,4S)-4-amino-5-cyclohexyl-3-hydroxypentanoyl-L-($N^{\epsilon}$-cyclohexyl)lysyl isobutylamide]

Boc-Phe-His-ACHPA-Lys(Cbz)-NHiBu is dissolved in a mixture of methanol(20 ml), cyclohexanone (2 ml) and AcOH(0.5ml), and is hydrogenated at 40 psi over Pd-C(10%) for 24 hours. The catalyst is filtered off and the filtrate is concentrated to dryness in vacuo. The crude material is purified on LH-20 using methanol giving the title product.

## Claims

1. A peptide of the formula:

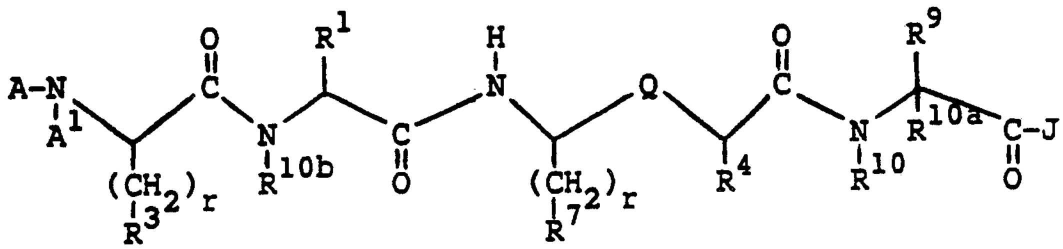 (I),

wherein:

A is hydrogen;

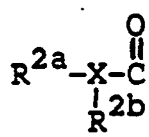 ,

where

X is -O-; -O-CH-; CH-O-; -CH-; N -; -NH-CH-; or -S-CH-; and $R^{2a}$ and $R^{2b}$ are independently hydrogen; unsubstituted or mono- or disubstituted $C_1$-$C_4$-alkyl, wherein the substituent(s) is/are independently chosen from the group consisting of amino; mono- or di-$C_1$-$C_4$-alkylamino; t-butoxycarbonylamino; carbobenzyloxyamino; acetylamino; trimethylacetylamino; t-butylacetylamino; hydroxy; $-SO_3H$; $-CO_2H$; $-CO_2$-$C_1$-$C_4$-alkyl; $C_1$-$C_4$-alkyl-$SO_2$; aryl, where aryl is unsubstituted or mono-, di- or trisubstituted phenyl or naphthyl, wherein the substituent(s) is/are independently selected from the group consisting of $C_1$-$C_8$-alkyl, phenyl-$C_1$-$C_4$-alkyl, amino, mono-or di-$C_1$-$C_4$-alkylamino, amino-$C_1$-$C_4$-alkyl, hydroxy-$C_1$-$C_4$-alkyl, mono- or di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$-alkyl, $CO_2$-$C_1$-$C_4$-alkoxy, $NR^5R^6$, and $N(R^5)_3^{\oplus}A^{\ominus}$, wherein $R^5$ and $R^6$ are independently hydrogen, unsubstituted or mono-substituted $C_1$-$C_4$-alkyl, where the substituent is amino, mono- or di-$C_1$-$C_4$-alkylamino, hydroxyl, $C_1$-$C_4$-alkoxy or $N(C_1\text{-}C_4\text{-alkyl})_3^{\oplus}A^{\ominus}$;

and $A^{\ominus}$ is a counterion selected from the group consisting of single negatively-charged ions; $-CONR^5R^6$, where $R^5$ and $R^6$ are as defined above; and Het, where Het is an unsubstituted or mono- or disubstituted 5- or 7- membered mono- or bicyclic or 7- to 10- membered bicyclic heterocyclic ring, wherein the one or two heteroatoms are independently selected from the group consisting of N, O, S, NO, SO, $SO_2$ and quaternized N, and the substituent(s) is/are independently selected from the group consisting of hydroxy, thiol, $C_1$-$C_6$-alkyl, $CF_3$, $C_1$-$C_4$-alkoxy, halo, aryl, as defined above, aryl-$C_1$-$C_4$-alkyl, amino, mono- or di-$C_1$-$C_4$-alkylamino, amino-$C_1$-$C_4$-alkyl, hydroxy-$C_1$-$C_4$-alkyl, di-$C_1$-$C_4$-alkylamino-$C_1$-$C_4$-alkyl, guanidyl, guanidyl-$C_1$-$C_4$-alkyl, $CO_2H$, $CO_2$-$C_2$-$C_4$-alkyl, $NR^5R^6$ and $N(R^5)_3^{\oplus}A^{\ominus}$, wherein $R^5$, $R^6$, and $A^{\ominus}$ are as defined above, or when the heteroatom N is present, the substituents are also alternatively $-(CH_2)_q-$,

wherein q is 3-to-6, or $-(CH_2)_2$-O-$(CH_2)_2$-and form a quaternary spirocyclic ring with the N-atom;

except that where X is -O-, only one of $R^{2a}$ or $R^{2b}$ is present;

$R^{2c}SO_2$-,

where

$R_{2c}$ is unsubstituted, mono- or di-substituted $C_1$-$C_4$ alkyl, wherein the substituent(s) is/are independently chosen from the group consisting of amino, mono- or di-$C_1$-$C_4$-alkylamino, t-butoxycarbonylamino, car-

bobenzyloxyamino, acetylamino, trimethylacetylamino, hydroxy, $-SO_3H$, $-CO_2H$, $-CO_2-C_1-C_4$-alkyl, $C_1-C_4$-alkyl-$SO_2$- and Het, as defined above; or

Het-CO, where Het is as defined above;

$A^1$ is hydrogen or $C_1-C_4$-alkyl;

r is 1-to-4;

$R^3$ is hydrogen, $C_1-C_4$-alkyl, aryl, as defined above, aryl-$C_1-C_4$-alkyl, or indolyl;

$R^1$ is hydrogen;

aryl, as defined above;

Het-S-$CH_2$;

Het-SO-$CH_2$;

Het-$SO_2CH_2$; or

unsubstituted or monosubstituted $C_1-C_4$-alkyl or $C_1-C_4$-alkylthio-$C_1-C_4$-alkyl, where the substituent is on the terminal carbon atom and is chosen from the group consisting of $C_1-C_4$-alkoxy, amino, mono- or di-$C_1-C_4$-alkylamino, carboxy, $CO_2-C_1-C_4$-alkyl, aryl as defined above, Het, as defined above, or $-CONR^5R^6$, wherein $R^5$ and $R^6$ are as defined bove;

$R^7$ is $C_3-C_6$-alkyl;

aryl, as defined above; or

unsubstituted or mono-, di- or trisubstituted $C_3-C_7$-cycloalkyl, where the substituent(s) is/are independently selected from the group consisting of $C_1-C_4$-alkyl, trifluoromethyl, hydroxy, $C_1-C_4$-alkoxy and halo;

Q is 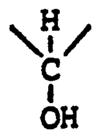 ; 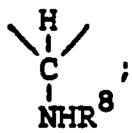 ; 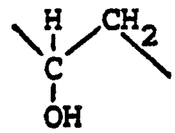 ; or 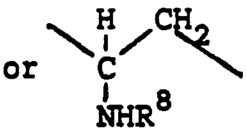 ,

where

$R^8$ is hydrogen; $C_1-C_4$-alkyl; formyl; $C_1-C_4$-alkanoyl; aroyl; $C_1-C_4$-alkoxycarbonyl; aryloxycarbonyl; or aryl-$C_1-C_4$-alkoxycarbonyl, wherein aryl is as defined above;

$R^4$ is hydrogen;

$C_2-C_8$-alkenyl;

mono- or disubstitued $C_2-C_8$-alkyl, where the substituent(s) is/are on the final 1 or/and 2 carbon atoms of the alkyl chain and is/are independently selected from the group consisting of hydroxy, carboxy, $CO_2-C_1-C_4$-alkyl, amino, mono- , di- or tri-$C_1-C_4$-alkylamino, guanidyl, $NR^5R^6$ and $N(R^5)_3^{\oplus}A^{\ominus}$, wherein $R^5$, $R^6$ and $A^{\ominus}$ are as defined above;

aryl, as defined above; or

unsubstituted, mono-, di- or trisubstituted $C_3$ -$C_7$-cycloalkyl, where the substituent(s) is/are independently selected from the group consisting of $C_1-C_4$-alkyl, trifluoromethyl, hydroxy, $C_1-C_4$-alkoxy and halo;

$R^9$ is $-(CH_2)_s-NR^{11}R^{12}$,

where

$R^{11}$ and $R^{12}$ are independently hydrogen, Het, as defined above, aryl, as defined above, $C_3-C_7$-cycloalkyl, or unsubstituted or monosubstituted $C_1-C_4$-alkyl, wherein the substituent is on the terminal carbon atom and is chosen from the group consisting of aryl, as defined above; Het, as defined above; amino; hydroxyl; mono- or di-$C_1-C_4$-alkylamino; $CO_2H$; $CONR^5R^6$, where $R^5$ and $R^6$ are as defined above; and $SO_3H$; and s is 1 or 2;

$-(CH_2)_tNR^{11a}R^{12a}$,

where

$R^{11a}$ and $R^{12a}$ are independently Het, as defined above, aryl, as defined above, $C_3-C_7$-cycloalkyl, or monosubstituted $C_1-C_4$-alkyl, wherein the substituent is on the terminal carbon atom and is chosen from the group consisting of aryl, as defined above;

Het, as defined above; amino; hydroxyl; mono- or di-$C_1-C_4$-alkylamino; $CO_2H$; $CONR^5R^6$, where $R^5$ and $R^6$ are as defined above; and $SO_3H$; and t is 3 or 4;

$-(CH_2)_uN(R^{11}R^{12}R^{13})^{\oplus}A^{\ominus}$,

where

u is 2-to-5; $R^{13}$ is independently chosen from the definitions of $R^{11}$ ; and $R^{11}$ and $A^{\ominus}$are as defined above;

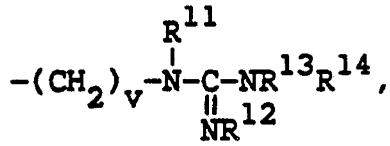

where

$R^{14}$ is independently chosen from the definitions of $R^{11}$, or together with $R^{13}$ is $-(CH_2)_4-$ or $-(CH_2)_5-$; v is 1-to-5; and $R^{11}$, $R^{12}$ and $R^{14}$ are as defined above;

$(CH_2)_s-R^{18}$,

where

$R^{18}$ is $-CH(CH_2CH_2)_2NR^{10}$

, 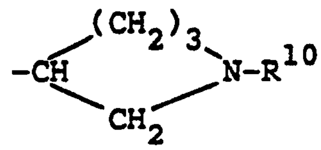 or

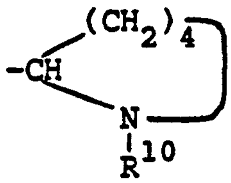 ,

wherein $R^{10}$ is H or $C_1$-$C_4$-alkyl; and s is as defined above; or

when either $R^{10}$ or $R^{10a}$ is $C_1$-$C_4$-alkyl, also alternatively $-(CH_2)_4-NR^{11}R^{12}$, wherein $R^{11}$ and $R^{12}$ are as defined above;

$R^{10a}$ and $R^{10b}$ are independently H or $C_1$-$C_4$-alkyl;

J is

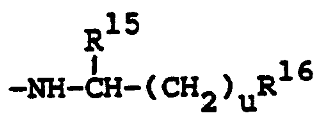

where $R^{15}$ is hydrogen, $C_1$-$C_4$-alkyl, or aryl-$C_1$-$C_4$-alkyl, wherein aryl is as defined above; $R^{16}$ is hydrogen; $-CO_2R^{17}$, wherein $R^{17}$ is hydrogen, $C_1$-$C_4$-alkyl, phenyl-$C_1$-$C_4$-alkyl or $C_5$-$C_{10}$-cycloalkyl; $CONR^{17}R^{18}$, wherein $R^{18}$ is independently selected from the definitions of $R^{17}$ and $R^{17}$ is as defined above; or, when u is not equal to 0, is also alternatively amino or mono- or di-$C_1$-$C_4$-alkylamino; and u is 0-to-4; or

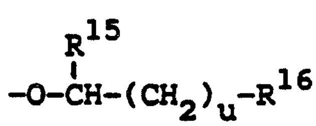

where $R^{15}$, $R^{16}$ and u are as defined above;

or a pharmaceutically-acceptable salt thereof.

2. A peptide according to Claim 1,

selected from the group consisting of:

Boc-Phe-His-ACHPA-HLys-NH-i-Bu

Boc-Phe-His-ACHPA-$NHCH[CH_2N(CH_3)_2]CO-NHCH_2CH_2NH_2$

Boc-Phe-His-ACHPA-[$N^{\epsilon}$-(pyridin-4-yl)]Lys-NH-2(S)-methylbutyl

Boc-Phe-His-ACHPA-$NHCH(CH_2NH$-i-Pr)$CO-NHCH_2CH_2OH$

Boc-Phe-His-ACHPA-($N^{\epsilon}$-$CH_2CO_2H$)Lys-$NHCH_2Ph$

Boc-Phe-His-ACHPA-$NH(CH_2NH_2)CO-NHCH$(2-butyl)$CH_2OH$

[Boc-Phe-His-ACHPA-($N^{\epsilon}$,$N^{\epsilon}$,$N^{\epsilon}$-trimethyl)Lys-$NHCH_2CH_2CO_2H]^{\oplus}Cl^{\ominus}$

Boc-Phe-His-ACHPA-($N^{\epsilon}$-benzyl)Lys-$NHCH_2CH_2CH_2OH$
Boc-Phe-His-ACHPA-($N^{\epsilon}$-benzyl)Lys-$NHCH_2CH_2CH_2CO_2H$
Boc Phe-His-ACHPA-($N^{\epsilon}$-benzyl)Lys-$NHCH_2CH_2CH_2CO_2Et$
Boc-Phe-His-ACHPA-($N^{\epsilon}$-benzyl)Lys-$NHCH_2CH_2CH_2CONEt_2$
Boc-Phe-His-ACHPA-[$N^{\epsilon}$-(piperidin-4-yl)]Lys-$NHCH_2Ph$
Boc-(p-OMe)Phe-His-ACHPA-HLys-NH-CH(2-butyl)$CH_2NH_2$
Boc-$NHCH_2CH_2OCO$-Phe-His-ACHPA-HLys-NHCH(2-butyl)-$CH_2N(CH_3)_2$ $(CH_3)_3CCH_2CO$-$NHCH_2CH_2OCO$-Phe-His-ACHPA-HLys-$NHCH_2CH_2CH_2NEt_2$
$(CH_3)_3CCH_2CO$-$NHCH_2CH_2OCO$-Phe-His-ACHPA-($N^{\alpha}$-methyl)Lys-$NHCH_2CH_2NH_2$
Boc-Phe-His-ACHPA-$NHCH[(CH_2)_4NHC(=NCH_3)NHCH_3]CO$-$NHCH_2Ph$
Boc-Phe-His-ACHPA-($N^{\epsilon}$-imidazolin-2-yl)Lys-NH-i-Bu
Boc-Phe-($N^{\alpha}$-Me)His-ACHPA-($N^{\epsilon}$-benzyl)Lys-$NHCH_2CH_2CH_2OH$
Boc-Phe-($N^{\alpha}$-Me)-His-ACHPA-($N^{\epsilon}$-benzyl)Lys-$NHCH_2CH_2CH_2CO_2H$
Boc-Phe-($N^{\alpha}$-Me)-His-ACHPA-($N^{\epsilon}$-benzyl)Lys-$NHCH_2CH_2CH_2$ $CO_2Et$
Boc-Phe-($N^{\alpha}$-Me)His-Cal[$CH(OH)CH_2$]Val-($N^{\epsilon}$-benzyl)Lys-$NHCH_2Ph$
Boc-Phe-($N^{\alpha}$-Me)His-Cal[$CH(NH_2)CH_2$]Val-HLys-$NHCH_2Ph$
Boc-Phe-($N^{\alpha}$-Me)His-Cal[$CH(OH)CH_2$]Val-$CH(CH_2NH_2)CO$-$N(CH_3)CH_2Ph$
Boc-Phe-($N^{\alpha}$-Me)His-Cal[$CH(OH)CH_2$]Val-$NHCH(CH_2CH_2NH_2)CO$-$NHCH_2Ph$
$HO_2C(CH_2)_4CO$-Phe-His-ACHPA-HLys-$NHCH_2Ph$
$HO_2C(CH_2)_4CO$-Phe-His-ACHPA-$NHCH[CH_2N(CH_3)_2]CO$-NH-n-Bu
$HO_2CCH_2C(CH_3)_2CO$-Phe-His-ACHPA-HLys-$NEt_2$
$O(CH_2CH_2)_2NCO$-Phe-His-ACHPA-($N^{\epsilon}$-benzyl)Lys-$NHCH_2CH_2OH$
$iPrSO_2$-Phe-His-ACHPA-($N^{\epsilon}$-benzyl)Lys-$NHCH_2CH_2CO_2H$
$(CH_3)_2N(CH_2)_3CO$-Phe-His-ACHPA-HLys-$NHCH_2Ph$
$O(CH_2CH_2)_2NCO$-($N^{\alpha}$-Me)Phe-His-ACHPA-HLys-NH-i-Bu
$CH_3N(CH_2CH_2)_2NCO$-Phe-His-ACHPA-$NHCH(CH_2NH_2)CO$-$NHCH_2CH_2CO_2H$
$SO_2(CH_2CH_2)_2NCO$-Phe-His-ACHPA-$NHCH(CH_2CH_2NH_2)CO$-$NHCH_2Ph$
$SO_2(CH_2CH_2)_2NCO$-($N^{\alpha}$-Me)Phe-His-ACHPA-$NHCH(CH_2CH_2NH_2)CO$-$NHCH_2Ph$
$HO_2CCH_2N(CH_2CH_2)_2NCO$-Phe-His-ACHPA-HLys-$NHCH_2CH_2Ph$
$HO_2CCH_2CH_2N(CH_2CH_2)_2NCO$-Phe-His-ACHPA-HLys-$NHCH_2Ph$
$HO_2CCH_2(CH_3)NCO$-Phe-His-ACHPA-HLys-$N(CH_3)CH_2Ph$
$O(CH_2CH_2)_2NCH_2CH_2SO_2$-Phe-His-ACHPA-HLys-$N(CH_3)CH_2Ph$
Boc-Phe-His-(2S-isobutyl)ACHPA-HLys-$NHCH_2CH(CH_3)CO_2H$
Boc-Phe-His-AmACHPA-HLys-$NHCH_2CH_2CH_2CO_2H$
Boc-Phe-His-ACHPA-($N^{\alpha}$-methyl)Lys-$NHCH_2CH_2CH_2OH$
Boc-Phe-His-ACHPA-($N^{\alpha}$-methyl-$N^{\epsilon}$-benzyl)Lys-$NHCH_2CH_2$ $CH_2CO_2H$
Boc-Phe-His-ACHPA-($N^{\alpha}$-methyl)Lys-$NHCH_2CH_2CH_2CO_2Et$
$HO_2CCH_2N(CH_2CH_2)_2NCO$-Phe-His-ACHPA-($\alpha$-methyl)Lys-$NHCH_2$ $CH_2Ph$
$HO_2CCH_2CH_2N(CH_2CH_2)_2NCO$-Phe-His-ACHPA-($\alpha$-methyl)Lys-$NHCH_2Ph$
$HO_2CCH_2(CH_3)NCO$-Phe-His-ACHPA-($\alpha$-methyl)Lys-$N(CH_3)$ $CH_2Ph$;
$O(CH_2CH_2)_2NCH_2CH_2SO_2$-Phe-His-ACHPA-($\alpha$-methyl)Lys-$N(CH_3)CH_2Ph$;
$O(CH_2CH_2)_2NCO$-Phe-His-ACHPA-($N^{\epsilon}$,$N^{\epsilon}$-dimethyl)Lys-$NHCH_2C_6H_5$; and
Boc-Phe-His-ACHPA-($N^{\epsilon}$-cyclohexyl)Lys-NH-iBu.

3. A peptide of the formula, A-B-D-G-T-J, wherein A is Boc, EtOC, isopropylsulfonyl, (4-carboxy) pentanoyl, (2-N-Boc-amino)ethyloxycarbonyl, or (morpholin-4-yl)carbonyl; B is Phe, HPhe, Trp or Tyr(OMe); D is His, Ala, Ile, Leu, Met, Nle, Nva or Phe; G is ACHPA, Sta, AmACHPA or HomoACHPA; T is Arg, HArg, Orn, Dap, Dab, HLys or ($N^{\alpha}$-methyl)Lys; and J is n-butyl, isobutyl or benzyl.

4. A pharmaceutical composition for renin associated hypertension or congestive heart failure comprising a pharmaceutical carrier and a therapeutically-effective amount of a peptide according to Claim 1.

5. A pharmaceutical composition according to Claim 4, also comprising an adjuvant.

6. A pharmaceutical composition according to Claim 4, also comprising one or more compounds selected from the group consisting of:

Diuretics:

acetazolamide, amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic

mixture, or as either the (+) or (-) enantiomer alone, or a manipulated ratio, e.g., 9:1 of said enantiomers, respectively); metolazone, methyclothiazide; muzolimine; polythiazide, quinethazone; sodium ethacrynate; sodium nitroprusside, spironolactone; ticrynafen; triamterene, trichlormethiazide;

α-Adrenergic Blocking Agents:

dibenamine; phentolamine; phenoxybenzamine; prazosin; tolazoline;

β-Adrenergic Blocking Agents:

atenolol; metoprolol; nadolol; propranolol; timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);
((±)-1-(4-((2-isopropoxyethoxy)methyl)phenoxy)-3-isopropylamino-2-propanol)fumarate) (bisoprolol);
(4-(2-hydroxy-3-[4-(phenoxymethyl)-piperidino]propoxy)-indole);
(carbazolyl-4-oxy-5,2-(2-methoxyphenoxy)-ethylamino-2-propanol);
(1-((1,1-dimethylethyl)amino)-3-((2-methyl-1H-indol-4-yl)oxy)-2-propanol benzoate) (bopindolol);
(1-(2-exobicyclo[2.2.1]-hept-2-ylphenoxy)-3-[(1-methylethyl)-amino]-2-propanol HCl) (bornaprolol);
(o-[2 hydroxy-3-[(2-indol-3-yl-1,1-dimethylethyl)amino]propoxy]benzonitrile HCl) (bucindolol);
(α-[(tert.butylamino)methyl]-7-ethyl-2-benzofuranmethanol) (bufuralol);
(3-[3-acetyl-4-[3-(tert.butylamino)-2-hydroxypropyl]phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3′-acetyl-4′-(2-hydroxy-3-isopropylaminopropoxy)acetanilide HCl) (diacetolol);
(methyl-4-[2-hydroxy-3-[(1-methylethyl)aminopropoxy]]benzenepropanoate HCl) (esmolol);
(erythro-DL-1-(7-methylindan-4-yloxy)-3-isopropylaminobutan 2-ol);
(1-(tert.butylamino)-3-[O-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2-hydroxy-5-[(R)-1-hydroxy-2-[(R)-(1-methyl-3-phenylpropyl)amino]ethyl]benzamide);
(4-hydroxy-9-[2-hydroxy-3-(isopropylamino)-propoxy]-7-methyl-5H furo[3,2-g][1]-benzopyran-5-one) (iprocrolol);
((-)-5-(tert.butylamino)-2-hydroxypropoxy]-3,4-dihydro-1-(2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropyl aminopropoxy)phenyl]ethyl-N′-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N′-(4′-chloro-2,3-dihydro-3-oxo-5-pyridazinyl)ethylenediamine);
((±)-N-[3-acetyl-4-[2-hydroxy-3-[(1-methylethyl)amino]propoxy]phenyl]butanamide) (acebutolol);
((±)-4′-[3-(tert-butylamino)-2-hydroxypropoxy]spiro[cyclohexane-1,2′-indan]-1′-one) (spirendolol);
(7-[3-[[2-hydroxy-3-[(2-methylindol-4-yl)oxy]propyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-(tert.butylamino)-2-hydroxy-propoxy)benzonitrile HCl) (bunitrolol);
((±)-2′-[3-(tert-butylamino)-2-hydroxypropoxy-5′-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbostyril HCl) (carteolol);
(1-(tert.butylamino)-3-(2,5-dichlorophenoxy)-2-propanol) (cloranolol);
(1-(inden-4(or 7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy-1-naphthyl)oxy]-2-propanol) (nadolol);
((S)-1-(2-cyclopentylphenoxy)-3-[(1,1-dimethylethyl)amino]-2-propanol sulfate (2:1)) (penbutolol);

(4′-[1-hydroxy-2-(amino)ethyl]methanesulfonanilide) (sotalol);
(2-methyl-3-[4-(2-hydroxy-3-tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-β-methylcinnamonitrile) (pacrinolol);
((±)-2-(3′-tert.butylamino-2′-hydroxypropylthio)-4-(5′-carbamoyl-2′-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);
(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);
(3,4-dihydro-8-(2-hydroxy-3-isopropylaminopropoxy)-3-nitroxy-2H-1-benzopyran) (nipradolol);

α- and β-Adrenergic Blocking Agents:

((±)-1-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol HCl);
(4-hydroxy-α-[[3-(4-methoxyphenyl)-1-methylpropyl]aminomethyl]-3-(methylsulfinyl)-benzmethanol HCl) (sulfinalol);
(5-[1-hydroxy-2-[[2-(o-methoxyphenoxy)ethyl]amino]ethyl]-2-methylbenzenesulfonamide HCl);
(5-[1-hydroxy-2-[(1-methyl-3-phenylpropyl)amino]ethyl]salicylamide HCl) (labetalol);
(1-((3-chloro-2-methyl-1H-indol-4-yl)oxy)-3-((2-phenoxyethyl)amino)-2-propanol-hydrogenmalonate) (ifendolol);
(4-(2-hydroxy-3-[(1-methyl-3-phenylpropyl)amino]propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)-3,4-dihydroxy)quinoxolin-2(1H)-one);

CNS-Acting Agents:

clonidine; methyldopa;

Adrenergic Neuron Blocking Agents:

guanethidine, reserpine and other rauwolfia alkaloids such as rescinnamine;

Vasodilators:

diazoxide; hydralazine; minoxidil;

Angiotensin I Converting Enzyme Inhibitors:

1-(3-mercapto-2-methyl-1-oxopropyl)-L-proline (captopril);
(1-(4-ethoxycarbonyl-2,4(R,R)-dimethylbutanoyl)indoline-2(S)-carboxylic acid);
(2-[2-[[1-(ethoxycarbonyl)-3-phenyl-propyl]amino]-1-oxopropyl]-1,2,3,4-tetrahydro-3-isoquinoline carboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)-cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl);

(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1-(S)-carboxy-3-phenylpropyl]-L-lysyl-L-proline (lisinopril);

Calcium Channel Blockers:

α-[3-[[2-(3,4-dimethoxyphenyl)ethyl]methylamino]propyl]-3,4-dimethoxy-α-(1-methylethyl)benzeneacetonitrile (verapamil);
1,4-dihydro-2,6-dimethyl-4-(2-nitrophenyl)-3,5-pyridinedicarboxylic acid dimethyl ester (nifedipine);
2-(2,2-dicyclohexylethyl)piperidine (perhexiline);
N-(1-methyl-2-phenylethyl)- -phenylbenzenepropanamine (prenylamine);
3-(aminosulfonyl)-4-chloro-N-(2,3-dihydro-2-methyl-1H-indol-1-yl)benzamide (indapamide);
(2′-(2-diethylaminoethoxy)-3-phenylpropiophenone (etafenone);
(4-[4,4-bis-(4-fluorophenyl)butyl]-N-(2,6-dimethylphenyl)-1-piperazineacetamide) (lidoflazine);
(2-(N-benzyl-N-methylamino)ethylmethyl-2,6-dimethyl-4-(m-nitrophenyl)-1,4-dihydro-3,5-pyridinedicarboxylate HCl) (nicardipine);
(N-(3,4-dimethoxyphenethyl)-2-(3,4-dimethoxyphenyl)-N-methyl-m-dithiane-2-propylamine-1,1,3,3-tetraoxide) (tiapamil);
(5,6-dimethoxy-2-(3-[(α-(3,4-dimethoxy)phenylethyl)methylamino]propyl)phthalimidine) (falipamil);
(β-[(2-methylpropoxy)methyl]-N-phenyl-N-phenylmethyl-1-pyrrolidineethanamine HCl monohydrate) (bepridil);
((+)-cis-3-(acetyloxy)-5-[2-(dimethylamino)ethyl]-2,3-dihydro-2-(4-methoxyphenyl)-1,5-benzothiazepin-4-(5H)-one) (diltiazem);
((E)-1-[bis-(p-fluorophenyl)methyl]-4-cinnamylpiperazine di HCl) (flunarizine);
(5-[(3,4-dimethoxyphenethyl)methylamino]-2-isopropyl-2-(3,4,5-trimethoxyphenyl)valeronitrile (gallopamil);
(ethylmethyl(2,3-dichlorophenyl)-1,4-dihydro-2,6-dimethyl-3,5-pyridinedicarboxylate (felodipine);
(isopropyl-2-methoxyethyl-1,4-dihydro-2,6-dimethyl-4-(3-nitrophenyl)-3,5-pyridinecarboxylate) (nimodipine);
(3-ethyl-5-methyl-1,4-dihydro-2,6-dimethyl-4-(3-nitrophenyl)-3,5-pyridine-dicarboxylate) (nitrendipine); and

Other Antihypertensive Agents:

aminophylline; cryptenamine acetates and tannates; deserpidine; meremethoxylline procaine; pargyline; trimethaphan camsylate; and the like, as well as admixtures and combinations thereof.

7. The use of a peptide as claimed in claim 1 for the preparation of a medicament useful for treating renin-associated hypertension or congestive heart failure in mammals.

8. The use as claimed in Claim 7, wherein mammals are human and the therapeutically-effective amount is from 0.02 to 10 grams per day.